# EUROPEAN PATENT SPECIFICATION

(11) **EP 1 644 519 B1**
(45) Date of publication and mention of the grant of the patent: **31.12.2008**
(21) Application number: 04737521.7
(22) Date of filing: 05.07.2004
(51) Int. Cl.: C12Q 1/68

(54) **METHOD FOR DETECTION OF ALKYLATED CYTOSINE IN DNA**
VERFAHREN ZUM NACHWEIS VON ALKYLIERTEM CYTOSIN IN DNA
PROCEDE POUR LA DETECTION DE CYTOSINE ALKYLEE DANS L'ADN

(30) Priority: 04.07.2003 AU 2003903430; 04.08.2003 US 491995 P
(43) Date of publication of application: 12.04.2006
(73) Proprietor: JOHNSON & JOHNSON RESEARCH PTY LIMITED, Eveleigh, NSW 1430 (AU)
(72) Inventor: TODD, Alison Velyian, Glebe, NSW 2037 (AU); FUERY, Caroline Jane, Toongabbie, NSW 2146 (AU); APPLEGATE, Tanya Lynn, Greenwich, NSW 2065 (AU)
(74) Representative: Brasnett, Adrian Hugh
(86) International application number: PCT/AU2004/000900
(87) International publication number: WO 2005/003381

(56) References cited:
- WO-A-20/05005660
- WO-A2-02/061124
- CA-A1- 2 462 928
- RAMIRO ALMUDENA R ET AL: "Transcription enhances AID-mediated cytidine deamination by exposing single-stranded DNA on the nontemplate strand." NATURE IMMUNOLOGY, vol. 4, no. 5, May 2003 (2003-05), pages 452-456, XP002302401 ISSN: 1529-2908
- PHAM P. ET AL.: 'Processive AID-catalyzed cytosine deamination on single-stranded DNA simulates somatic hypermutation' NATURE vol. 424, no. 6944, July 2003, pages 103 - 107, XP002302398
- BRANSTEITTER R. ET AL.: 'Activation-induced cytidine deaminase deaminates deoxycytidine on single-stranded DNA but requires the action of RNase' PROCEEDINGS OF THE NATIONAL ACADEMY OF SCIENCES USA vol. 100, no. 7, April 2003, pages 4102 - 4107, XP002302399
- PETERSEN-MAHRT S.K.: 'In vitro deamination of cytosine to uracil by apolipoprotein B editing complex catalytic subunit 1 (APOBEC1)' JOURNAL OF BIOLOGICAL CHEMISTRY vol. 278, no. 22, May 2003, pages 19583 - 19586, XP008063736
- REIN T. ET AL.: 'Identifying 5-methylcytosine and related modifications in DNA genomes' NUCLEIC ACID RESEARCH vol. 26, no. 10, 1998, pages 2255 - 2264, XP002143106
- CLARK S.J. ET AL.: 'High sensitivity mapping of methylated cytosines' NUCLEIC ACID RESEARCH vol. 22, no. 15, 1994, pages 2990 - 2997, XP002210107

### SEQUENCE LISTING

<110> Johnson & Johnson Research Pty Limited
<120> Method for Detection of Alkylated Cytosine in DNA
<130> AHB/FP6349021
<140> EP 04737521.7
   <141> 2004-07-05
<150> PCT/AU2004/000900
   <151> 2004-07-05
<150> AU 2003903430
   <151> 2003-07-04
<150> US 60/491,995
   <151> 2003-08-04
<160> 14
<170> PatentIn version 3.3
<210> 1
   <211> 80
   <212> DNA
   <213> Artificial
<220>
   <223> Oligonucleotide Ecad80
<400> 1
   cgctgctgat tggctgtggc cggcaggtga accctcagcc aatcagcggt acggggggcg      60
   gtgctccggg gctcacctgg                                                  80
<210> 2
   <211> 27
   <212> DNA
   <213> Artificial
<220>
   <223> Oligonucleotide AA1
<400> 2
   tgttttgggt gtgtatggtt tgggtgt 27
<210> 3
   <211> 27
   <212> DNA
   <213> Artificial
<220>
   <223> Oligonucleotide AA2
<400> 3
   acacccaaac catacacacc caaaaca 27
<210> 4
   <211> 80
   <212> DNA
   <213> Artificial
<220>
   <223> Oligonucleotide Ecad80M3
<220>
   <221> misc_feature
   <222> (21)..(21)
   <223> where 'n' is 5'-methylcytosine
<220>
   <221> misc_feature
   <222> (47)..(47)
   <223> where 'n' is 5'-methylcytosine
<220>
   <221> misc_feature
   <222> (52)..(52)
   <223> where 'n' is 5'-methylcytosine
<400> 4
   cgctgctgat tggctgtggc nggcaggtga accctcagcc aatcagnggt anggggggcg 60
   gtgctccggg gctcacctgg 80
<210> 5
   <211> 36
   <212> DNA
   <213> Artificial
<220>
   <223> Oligonucleotide T1
<400> 5
   attatattta aatatataaa atatatatta ataaat 36
<210> 6
   <211> 36
   <212> DNA
   <213> Artificial
<220>
   <223> Oligonucleotide T2
<400> 6
   atttattaat atatatttta tatatttaaa tataat 36
<210> 7
   <211> 80
   <212> DNA
   <213> Artificial
<220>
   <223> Oligonucleotide antisense sequence of Ecad80 (ASEcad80)
<400> 7
   ccaggtgagc cccggagcac cgccccccgt accgctgatt ggctgagggt tcacctgccg 60
   gccacagcca atcagcagcg 80
<210> 8
   <211> 22
   <212> DNA
   <213> Artificial
<220>
   <223> Chemically synthesised looping probe DNA sequence LP10
<400> 8
   cgaccgcccc gattggctga gg 22
<210> 9
   <211> 23
   <212> DNA
   <213> Artificial
<220>
   <223> Chemically synthesised looping probe DNA sequence LP26
<400> 9
   gccccggagc gagggttcac ctg 23
<210> 10
   <211> 24
   <212> DNA
   <213> Artificial
<220>
   <223> Chemically synthesised looping probe DNA sequence LP26+1
<400> 10
   gccccggagc ggagggttca cctg 24
<210> 11
   <211> 26
   <212> DNA
   <213> Artificial
<220>
   <223> Antisense oligonucleotide AS26
<400> 11
   agccaatcag cggtacgggg ggcggt 26
<210> 12
   <211> 18
   <212> DNA
   <213> Artificial
<220>
   <223> Substrate
<400> 12
   cggtacgggg ggcggtgg 18
<210> 13
   <211> 9
   <212> DNA
   <213> Artificial
<220>
   <223> Primer
<400> 13
   ccaccgccc 9
<210> 14
   <211> 18
   <212> DNA
   <213> Artificial
<220>
   <223> Substrate
<400> 14
   cggtaygggg ggcggtgg 18

### SEQUENCE LISTING

<110> Johnson & Johnson Research Pty Limited
<120> Method for Detection of Alkylated Cytosine in DNA
<130> AHB/FP6349021
<140> EP 04737521.7
   <141> 2004-07-05
<150> PCT/AU2004/000900
   <151> 2004-07-05
<150> AU 2003903430
   <151> 2003-07-04
<150> US 60/491,995
   <151> 2003-08-04
<160> 14
<170> PatentIn version 3.3
<210> 1
   <211> 80
   <212> DNA
<213> Artificial
<220>
   <223> Oligonucleotide Ecad80
<400> 1
   cgctgctgat tggctgtggc cggcaggtga accctcagcc aatcagcggt acggggggcg 60
   gtgctccggg gctcacctgg 80
<210> 2
   <211> 27
   <212> DNA
   <213> Artificial
<220>
   <223> Oligonucleotide AA1
<400> 2
   tgttttgggt gtgtatggtt tgggtgt 27
<210> 3
   <211> 27
   <212> DNA
   <213> Artificial
<220>
   <223> Oligonucleotide AA2
<400> 3
   acacccaaac catacacacc caaaaca 27
<210> 4
   <211> 83
   <212> DNA
   <213> Artificial
<220>
   <223> Oligonucleotide Ecad80M3
<400> 4
<210> 5
   <211> 36
   <212> DNA
   <213> Artificial
<220>
   <223> Oligonucleotide T1
<400> 5
   attatattta aatatataaa atatatatta ataaat 36
<210> 6
   <211> 36
   <212> DNA
   <213> Artificial
<220>
   <223> Oligonucleotide T2
<400> 6
   atttattaat atatatttta tatatttaaa tataat 36
<210> 7
   <211> 80
   <212> DNA
   <213> Artificial
<220>
   <223> Oligonucleotide antisense sequence of Ecad80 (ASEcad80)
<400> 7
   ccaggtgagc cccggagcac cgccccccgt accgctgatt ggctgagggt tcacctgccg 60
   gccacagcca atcagcagcg 80
<210> 8
   <211> 22
   <212> DNA
   <213> Artificial
<220>
   <223> Chemically synthesised looping probe DNA sequence LP10
<400> 8
   cgaccgcccc gattggctga gg 22
<210> 9
   <211> 23
   <212> DNA
   <213> Artificial
<220>
   <223> Chemically synthesised looping probe DNA sequence LP26
<400> 9
   gccccggagc gagggttcac ctg 23
<210> 10
   <211> 24
   <212> DNA
   <213> Artificial
<220>
   <223> Chemically synthesised looping probe DNA sequence LP26+1
<400> 10
   gccccggagc ggagggttca cctg 24
<210> 11
   <211> 26
   <212> DNA
   <213> Artificial
<220>
   <223> Antisense oligonucleotide AS26
<400> 11
   agccaatcag cggtacgggg ggcggt 26
<210> 12
   <211> 18
   <212> DNA
   <213> Artificial
<220>
   <223> Substrate
<400> 12
   cggtacgggg ggcggtgg 18
<210> 13
   <211> 9
   <212> DNA
   <213> Artificial
<220>
   <223> Primer
<400> 13
   ccaccgccc 9
<210> 14
   <211> 18
   <212> DNA
   <213> Artificial
<220>
   <223> Substrate
<400> 14
   cggtaygggg ggcggtgg 18

## Description

### FIELD OF THE INVENTION

The present invention relates to methods for detecting alkylated cytosine in DNA.
Methods of the invention employ enzymes that differentially modify alkylated cytosine and cytosine. The presence of alkylated cytosine in DNA is determined by evaluating the level of enzymatic modification of the DNA following incubation of the DNA with at least one such enzyme. The detection of alkylated cytosine in DNA is useful for diagnostic and other purposes.

### BACKGROUND OF THE INVENTION

At least seven different covalent base modifications have been identified in prokaryotic, eukaryotic, bacteriophage and/or viral genomes (1). In higher order eukaryotes the most abundant covalently modified base is 5-methylcytosine located 5' to guanosine in CpG dinucleotides. It has been hypothesised that methylation patterns play a role in gene transcription, X chromosome inactivation, genomic imprinting, cell differentiation and tumourigenesis (2).

The abnormal phenotype of cancer cells is due to qualitative and/or quantitative change. Sequence-based qualitative changes (genetic mutations) are preserved in the genomic DNA and this has facilitated their detection and characterisation. The inheritance of information on the basis of gene expression is known as epi-genetics. Methylation of cytosine bases in nucleic acid can effect epigenetic inheritance by altering expression of genes and by transmission of DNA methylation patterns through cell division. Cancer cells have been frequently shown to harbour both genetic and epi-genetic mutations.

Neoplastic cells simultaneously harbour multiple abnormalities relating to methylation patterns. They frequently have both widespread genomic hypomethylation as well as more regional areas of hypermethylation (1). Regional methylation of normally unmethylated CpG islands located within promoter regions of genes has been shown to be correlated with the down regulation of the corresponding gene. This hypermethylation can serve as an alternative mechanism to coding region mutations for the inactivation of tumour suppressor genes. Examples of genes which have CpG island hypermethylation in association with human tumours include p16 (lung, breast, colon, prostate, renal, liver, bladder, and head and neck tumours), *E-cadherin* (breast, prostate, colon, bladder, liver tumours), the von-Hippel Lindau (VHL) gene (renal cell tumours), *BRCA1* (breast tumours), p15 (leukemias, Burkitt lymphomas), hMLH1 (colon), ER (breast, colon, lung tumours; leukemias), HIC1 (brain, breast, colon, renal tumours), MDG1 (breast tumours), GST-π (prostate tumours), O⁶-MGMT (brain tumours), calcitonin (carcinoma, leukemia),and *myo-D*(bladder tumours) (1, 3).

The converse situation has also been reported, whereby CpG hypomethylation is thought to contribute to neoplastic progression. For instance, the urokinase CpG island was found to be hypermethylated in early stage, non-metastatic breast tumour cells but was hypomethylated in highly metastatic breast tumor cells (4). Similarly, hypomethylation of a region within the metastasis-associated S100A4 gene has been hypothesized as the mechanism of gene activation in colon adenocarcinoma cell lines (5).

At least eight different methods, along with several variations, allow characterisation of 5-methylcytosine or related modified bases in DNA genomes (2). Each method has advantages and disadvantages in terms of specificity, resolution, sensitivity and potential artefacts.

The total nucleic acid base composition of a genome can be determined by hydrolysing DNA to its constituent nucleotides, either chemically or enzymatically, and then fractionating and analysing the composition by standard methods (chromatography, electrophoresis and high pressure liquid chromatography). This approach quantifies the amount of modified bases present in the genome, but does not give any information on which part of the genome was originally modified. Dinucleotide composition and frequency can be determined by nearest-neighbour analysis, but again this method produces only limited sequence information. Neither of these methods are genome specific, and contamination of samples by DNA from viruses and other endoparasites can lead to misleading results.

More specific methods exist which can provide data on exactly where in the sequence of the genome modified bases exist. Genomic DNA can be analysed by restriction enzymes that are sensitive to methylation. With this method, however, the number of sites that can be examined is limited by the number of sequences recognized by methylation sensitive restriction enzymes. Sequencing would provide sequence-specific information, but methylation patterns are not preserved during PCR or when eukaryotic DNA is amplified in bacteria through molecular cloning.

It is necessary to differentially modify the bases, in a methylation specific manner, to produce a modified sequence where the methylation-specific changes are retained during sequencing protocols. There are currently three protocols that rely on analysis of differential base modification. All of these protocols involve modification of DNA, induced by chemical treatment of the DNA followed by analysis of the DNA sequence.

Hydrazine (N₂H₄), permanganate (MnO₄⁻), and bisulfite (HSO₃⁻) all differentially modify cytosine bases in genomic DNA depending on the methylation status of the cytosine base.

Hydrazine has a lower reactivity with 5-methylcytosine than with cytosine or thymine. After incubation of DNA with hydrazine the DNA is run on a sequencing gel. Comparison of the hydrazine-treated DNA with DNA treated with other base-specific chemical cleavage compounds allows the sequence of the DNA to be determined. In hydrazine-treated DNA samples 5-methylcytosine-containing sequence positions produce an absence or reduced intensity of bands compared to the cytosine and cytosine + thymidine specific ladders of sequencing reactions from genomic DNA. Thus the hydrazine protocol produces a negative result that correlates with the presence of 5-methylcytosine. Unambiguous identification of 5-methylcytosine requires the generation of a positive signal. A further disadvantage of hydrazine modification for the identification of 5-methylcytosine is that µg of template DNA is required.

Potassium permanganate, at weakly acidic pH and room temperature, reacts preferentially with thymine and 5-methylcytosine, and only weakly with cytosine and guanine. After incubation of DNA with permanganate the DNA is run on a sequencing gel. Comparison of the permanganate-treated DNA with DNA treated with other base-specific chemical cleavage compounds allows the sequence of the DNA to be determined. Permanganate oxidation of DNA can therefore be used to discriminate between cytosine and 5-methylcytosine (6). Although the permanganate protocol produces a positive result, and thus has an advantage over the hydrazine protocol, permanganate does react weakly with cytosine and hence discrimination of cytosine versus 5-methylcytosine depends on a difference in the intensities of their bands on the sequencing gel. A further disadvantage of permanganate modification is that µg of template DNA is again required.

Bisulfite treatment of genomic DNA deaminates unmethylated cytosine bases in the nucleic acid template to uracil, whereas 5-methylcytosine is resistant to deamination. Bisulfite has little activity on cytosine bases in double stranded DNA and so genomic double stranded DNA is preferably denatured to single stranded DNA. The standard bisulfite modification protocol uses incubation in alkali (NaOH) to denature double stranded DNA to single stranded DNA (7). Bisulfite deaminates cytosine slowly and incubation times have to achieve a compromise between complete deamination of all cytosine and fragmentation of DNA after long incubations. Protocols for bisulfite modification use a range of incubation times, generally from 4 to 20 hours incubation in bisulfite.

Grunau *et al* (8) studied optimum conditions for bisulfite-mediated deamination of cytosine and found that 4 hours incubation at 55°C gave 99% deamination of cytosine, but under these conditions 84 to 96% of the DNA was degraded, reducing yields for subsequent steps. Further, 5-methylcytosine is deaminated by heat at a greater rate than cytosine. For example, the rate of deamination of 5-methylcytosine at 60°C is 1.5 times higher than that of cytosine (9). Incubations in bisulfite at lower temperatures reduce fragmentation of DNA but the incubation times have to be extended to 14 to 20 hours to achieve full deamination of cytosines. Bisulfite modification requires approximately 10 ng of DNA for subsequent analysis using PCR-based methods.

The modified DNA sense and anti-sense strands produced by bisulfite modification are no longer complementary and therefore subsequent amplification by PCR must be performed with primers that are designed to be strand specific that is, the primers are complementary to either the modified sense strand or the modified anti-sense strand. When the region of interest is amplified by PCR, uracil (previously cytosine) is converted to thymine and 5-methylcytosine is converted to cytosine (7). The PCR products (amplicons) can be subsequently analysed by standard DNA sequencing (7) or other PCR-based techniques that produce sequence information such as methylation-specific PCR (10) or REMS-PCR (36), and analysis with restriction enzymes (3) or methylation-specific probes (11).

Although the bisulfite method has advantages in terms of ease of use and sensitivity over other existing protocols, potential artefacts can arise from the experimental protocol (2) namely not all cytosines are converted to uracil, a small percentage of 5-methylcytosine is converted to thymidine (12) (DNA polymerases do not distinguish between uracil and thymine) and there can be a loss of DNA from fragmentation caused by the long incubations and non-physiological buffers required (8). The full protocol is long and laborious involving 2 to 3 days of manipulation and at least 4 to 20 hours of incubation in bisulfite before results are obtained. The rate-limiting step in all epigenetic studies is sample preparation using the bisulfite modification protocol.

DNA extracted from many types of specimens including normal and tumour tissue, paraffin embedded tissues, as well as plasma and serum has been shown to contain aberrantly methylated sequences using the combination of bisulfite treatment and analysis by PCR-based methods (4,13,14).

A variety of enzymes with the ability to deaminate cytosine bases have been described. Cytidine Deaminase (EC 3.5.4.5.) converts cytidine to uridine and is widely distributed in prokaryotes and eukaryotes. Cytosine Deaminase (EC 3.5.4.1.) converts cytosine to uracil. Deoxycytidine Deaminase (EC 3.5.4.14.) converts deoxycytidine to deoxyuridine and Deoxycytidilate Deaminase converts deoxycytidine-5-phosphate to deoxyuridine-5-phosphate. These enzymes show different degrees of substrate specificity depending on the source of the enzyme. The ability of Cytidine Deaminase and Cytosine Deaminase to discriminate between 5-methylcytidine and 5-methylcytosine and their unmethylated analogues as substrates (respectively) is species specific. Cytidine Deaminase from humans can deaminate, with varying efficiency, numerous cytidine derivatives including cytosine, deoxycytidine, and 5-methylcytidine (15,16). Cytosine Deaminase from *Pseudomonas* can utilise 5-methylcytosine (17) while the enzyme produced by enterics can only use cytosine as a substrate. Cytosine Deaminase from the fungus *Aspergillus fumigatus* and the yeast enzyme can utilise 5-methylcytosine as a substrate (18,19).

Apolipoprotein B mRNA Editing Enzyme (ApoBRe) is the central component of an RNA editsome whose physiological role is specifically to deaminate the cytosine base at position #6666 of the *apoB* mRNA to uracil in gastrointestinal tissues creating a premature stop codon (20, 21). The catalytic component with cytidine deaminase activity is called Apolipoprotein B mRNA Editing Enzyme Catalytic Polypeptide 1 (APOBEC1). Although mRNA is the physiological substrate of this enzyme there is some evidence that it has activity on DNA *in vivo.* Misexpression of Apolipoprotein B mRNA Editing Enzyme in transgenic mice predisposes to cancer (22) and expression of human Apolipoprotein B mRNA Editing Enzyme in *E. coli* results in a mutator phenotype where there is a several 1000-fold enhanced mutation frequency seen at various loci in UNG-deficient strains.

UNG is an enzyme involved in the repair of U:G mismatches caused by spontaneous cytosine deamination and deficiency in this enzyme prevents cells from repairing deaminated cytosines in their genome (23). Sequencing of DNA showed that mutations were triggered by conversion of cytosine to uracil in DNA. There appears to be some context specificity in the small stretches of DNA studied in this model (23) with a requirement for a 5' flanking pyrimidine. This is despite that fact that the cytosine base (#6666) exclusively targeted for deamination by this enzyme in the physiological RNA substrate has a 5'flanking purine (adenosine). Deamination of cytosines with 5'flanking pyrimidines by Apolipoprotein B mRNA Editing Enzyme may require factors not supplied in the *E. coli* model.

Recent work by Petersen-Mahrt & Neuberger (24) investigated the deamination activity of Apolipoprotein B mRNA Editing Enzyme *in vitro* on DNA substrates. They found no activity on double stranded DNA but cytosine bases in chemically synthesized single stranded DNA substrates were readily deaminated with 57% deamination of three cytosine bases in 120 minutes of incubation with a crude extract of enzyme. The activity of the enzyme appeared to be slightly higher when treated with RNase. The authors calculated that one molecule of Apolipoprotein B mRNA Editing Enzyme in their crude extract could deaminate a single cytosine base in a chemically synthesised single stranded DNA substrate in 10 minutes. They attributed this slow rate of deamination to the fact that their assay was likely to be sub-optimal. This was attributed to the lack of other factors required for activity that were not expressed in the *E. coli* host, that the human enzyme might not properly fold in the *E. coli* host, and the fact that any post-translation modifications required for activity would not be supplied by the *E. coli* host.

Activation-Induced Cytidine Deaminase (known as AID or AICDA) is a B-cell specific protein. Expression of Activation-Induced Cytidine Deaminase is a pre-requisite to class-switch recombination, a process mediating isotype switching of immunoglobulin, and somatic hypermutation, which involves the introduction of many point mutations into the immunoglobulin variable region genes. The mode of action of Activation-Induced Cytidine Deaminase is unknown. Current theories focus on the fact that Activation-Induced Cytidine Deaminase has sequence motif homology with Apolipoprotein B mRNA-Editing Enzyme and Cytidine Deaminase.

An early theory on the mode of action of Activation-Induced Cytidine Deaminase suggested that the hypothesised RNA-editing function of the enzyme might be involved in editing mRNAs that encode proteins essential for class-switch recombination and somatic hypermutation. The theory with most experimental support suggests that Activation-Induced Cytidine Deaminase functions as a DNA-specific cytidine deaminase. This model suggests that Activation-Induced Cytidine Deaminase deaminates cytosine bases in somatic hypermutation hotspot sequences to produce G:U mismatches and that these are differentially resolved to effect somatic hypermutation or class switch recombination (25). Evidence for the latter theory includes the suggestion that somatic hypermutation is initiated by a common type of DNA lesion, and that there is a first phase of hypermutation that is specifically targeted to dC/ dG pairs. This would require Activation-Induced Cytidine Deaminase to have cytidine deaminase activity on DNA. All published work on Activation-Induced Cytidine Deaminase has focused on determining the *in vivo* substrate to elucidate the role of the enzyme in somatic hypermutation and isotype switching of immunoglobulin.

Research by various laboratories has showed that human Activation-Induced Cytidine Deaminase can deaminate cytosine on single stranded DNA *in vitro* (26-29) but not on single stranded RNA (26, 27). Activity of Activation-Induced Cytidine Deaminase on double-stranded DNA *in vitro* is limited to DNA coupled to transcription factors. It has been hypothesised that transcription allows deamination of double stranded DNA by generating secondary structures that provide single-stranded DNA substrates such as stable R loops and stem loops (28). These secondary structures can be mimicked *in vitro* by producing bubbles, or loops, of centrally located noncomplementary regions of DNA, which will be single stranded, between complementary regions of double stranded DNA. Activation-Induced Cytidine Deaminase deaminates cytosines in such bubbles. The efficiency of deamination depends on the length of the single stranded bubble. Bransteitter *et al.* (27) measured the percent of a chemically synthesised double stranded DNA substrate deaminated in 5 minutes of incubation and showed that substrates with 1 nucleotide bubbles were not deaminated, 3 nucleotide bubbles showed 5% deamination, 4 nucleotide bubbles showed 8 % deamination, 5 nucleotide bubbles showed 35 % deamination and 9 nucleotide bubbles showed 56 % deamination.

It has been hypothesised that Activation-Induced Cytidine Deaminase activity would be restricted to the physiological target (the immunoglobulin loci) because rampant DNA deaminase activity would be harmful to the cell. There is some suggestion that the deaminase activity of Activation-Induced Cytidine Deaminase is sequence specific (30), and it is hypothesised that Activation-Induced Cytidine Deaminase would show greatest activity on the somatic hypermutation hot-spot sequence RGYW (a sequence commonly mutated in the variable region of the immunoglobulin gene). Bransteitter *et al.* (27) showed that *in vitro* Activation-Induced Cytidine Deaminase had approximately three-fold higher activity on two hot-spot sequences compared with non-hot-spot sequences. Conversely, Dickerson *et al.* (26) found that the deaminase activity of Activation-Induced Cytidine Deaminase was sequence specific, but that cold-spot sequences (sequences of the variable region of the immunoglobulin gene that have never been found to be mutated *in vivo*) were deaminated equally well as hot-spot sequences, and that some hot-spot sequences were deaminated at only background levels.

Work by Pham *et al.* (31) tested the ability of Activation-Induced Cytidine Deaminase to deaminate cytosine bases *in vitro* using a large single stranded DNA template. In these experiments, the single stranded DNA template was a phage circular DNA substrate containing a 230-nucleotide target of the *lacZa* reporter sequence as part of a 365-nucleotide single-stranded gapped region. Incubations were carried out with 500 ng of the double-stranded phage DNA substrate with a 40-fold excess of enzyme in a 10 mM TRIS buffer (pH 8.0) with 1 mM EDTA and 1 mM dithiothreitol at 37°C for 20 minutes. The spectra of mutations were assessed by transfecting mutated phage (which gave white or light blue plaques) into UNG-deficient *E. coli* with subsequent sequencing of clones. Under the test conditions used the deamination activity of Activation-Induced Cytidine Deaminase was found to vary with sequence context, and the authors hypothesised that their results suggested the enzyme was a mobile molecule that processively deaminated cytosine molecules in the single stranded DNA.

Pham *et al.* (31) also described a protocol for measuring the deaminiation activity of Activation-Induced Cytidine Deaminase in a trancriptionally active version of their Phage substrate. Incubations were carried out with 30 nM of the double-stranded phage DNA substrate in a 50 mM HEPES buffer (pH 7.5) with 1 mM EDTA and 10 mM MgCl2 at 37°C for 30 minutes. The incubations included T7 RNA polymerase and rNTPs to produce transcriptionally active DNA which is a more accessible substrate for the Activation-Induced Cytidine Deaminase (27). These incubations showed that deamination mediated by Activation-Induced Cytidine Deaminase on the non-transcribed strand required RNA polymerase (active transcription) and that deamination on the transcribed strand, "protected" as an RNA-DNA hybrid, occurs at an approximately 15-fold lower rate. These incubations also demonstrated favoured deamination occurred in hotspot motifs.

Models that involve ectopic expression of Activation-Induced Cytidine Deaminase *in vivo* show untargeted cytosine deamination, that is deamination of genes other than the variable region of the immunoglobulin gene. For example, human Activation-Induced Cytidine Deaminase expressed in *E. coli,* which obviously lacks the human immunoglobulin target gene, produces context specific deaminations in genes screened for mutations (30). The reason for this context specific deamination was not examined.

Bransteitter *et al.* (27) recently incubated human Activation-Induced Cytidine Deaminase with a variety of chemically synthesized nucleic acid substrates *in vitro.* This work showed that, in a very simple model, Activation-Induced Cytidine Deaminase was capable of deaminating cytosine bases with 10-fold higher specific activity than 5-methylcytosine bases. The model involved incubating Activation-Induced Cytidine Deaminase with chemically synthesized single stranded DNA molecules with either 27 or 33 nucleotides, including either 1 or 2 cytosine bases, with no complimentary DNA strand present. These artificial substrates were present in high concentration, 100 nM, in a two-fold excess of Activation-Induced Cytidine Deaminase. The ability of Activation-Induced Cytidine Deaminase to differentially convert cytosine bases to uracil, with no or little activity on 5-methylcytosine, in a complex mixture of genomic DNA extracted from an individual where there are a multiplicity of mega-base fragments with a multiplicity of different sequence contexts of cytosine bases with both sense and complementary antisense strands present was neither tested nor considered.

The deaminase activity of Activation-Induced Cytidine Deaminase is inhibited by 1,10-phenanthroline, a strong chelator, but not by EDTA, a weaker chelator. This suggests that Activation-Induced Cytidine Deaminase requires a tightly bound metal ion, possibly zinc, for deaminase activity (27, 29). Activation-Induced Cytidine Deaminase retains deaminase activity over salt levels of 50 to 150 mM, can tolerate moderate levels of EDTA (5 to 10 mM), works at a wide range of pH (from 7.6 to 9.0 were tested) and works with varying efficiencies from room temperature to 37°C (26). These conditions are conducive to retaining the integrity of genomic DNA without fragmentation. Activation-Induced Cytidine Deaminase is still active after being heated at 65°C for 30 minutes (26).

Mutant forms of enzymes can exist in nature (e.g. allelic variants and forms arising from *in vivo* mutations) or can be artificially generated. Methods for generating mutant proteins are known in the art (39). Mutations can be artificially generated either following a rational approach, such as where specific amino acid substitutions, deletions or additions are generated, or they can be randomly generated, and the mutant form of the protein tested for the desired activity.

Enzymes which modify DNA require only a few hours incubation. Purified restriction enzymes, for example, require only 1 hour incubation in optimal conditions to fully cleave double stranded DNA. Bransteitter *et al.* (27) measured 95 % conversion of cytosine to uracil by Activation-Induced Cytidine Deaminase in a chemically synthesized single-stranded DNA substrate in 16 minutes, and 56 % conversion of cytosine to uracil in a synthetic substrate with a 9 nucleotide single stranded bubble after 5 minutes. This is thus a fast reaction. However, work by other groups, with different reaction conditions, have shown that only 10 % of a chemically synthesized single stranded DNA substrate containing one cytosine was converted to uracil after 30 minutes of incubation with Activation-Induced Cytidine Deaminase (26).

### SUMMARY OF THE INVENTION

In one aspect of the present invention there is provided a method for detecting the presence or level of alkylated cytosine in a sample of genomic or mitochondrial double stranded DNA from an individual, the method comprising:
(a) converting at least one region of the double stranded DNA to single stranded DNA;
(b) reacting a target region of the single stranded DNA from step (a) with at least one enzyme having cytidine deaminase activity, the enzyme differentially modifying alkylated cytosine and cytosine; and
(c) determining the level of enzymatic modification of the target region by the enzyme.

Generally, the reaction conditions under which the enzyme is used will be such that the enzyme reacts substantially only with either alkylated cytosine or cytosine but not both.

Preferably, the enzyme will be capable of reacting substantially with only one of alkylated cytosine or cytosine.

Preferably, the conversion of the region of the double stranded DNA to single stranded DNA will comprise at least partially separating the two strands. Separation of the strands may for instance be achieved by heat denaturation of the DNA or the use of strand displacement probes. Other techniques that may be employed include chemical or enzymatic denaturation of the double stranded DNA. The method may also comprise inhibiting annealing of the two strands of the double stranded DNA together once they have been separated to facilitate access to the target region of the single stranded DNA by the enzyme.

One or more probes capable of hybridising with a respective strand of the double stranded DNA may be utilised to inhibit annealing of the separated strands. When a plurality of probes are used, the probe(s) may hybridise with only one of the strands, or one or more of the probes may hybridise with one strand and the remaining probe or probes with the other strand.

Accordingly, a method of the invention may further comprise hybridising at least one probe with a strand of the double stranded DNA following separation of the two strands to inhibit annealing of the strands together and thereby facilitate access to the target region of the single stranded DNA by the enzyme.

The or each probe will normally be an oligonucleotide and may be selected from the group consisting of sense probes, looping probes for forming a loop in the single stranded DNA for access of the enzyme to the target region, antisense probes, and combinations thereof. More generally, a probe may hybridise with a single contiguous region of a strand of the double stranded DNA, or separate discrete upstream and downstream regions of the strand which flank the target region of the strand being evaluated for the presence or level of alkylated cytosine.

In the former instance, at least two such probes may be utilised, wherein one of the probes hybridises with a region of the strand downstream of the target region, and a further of the probes hybridises with the strand upstream of the target region such that hybridisation of the other strand of the double stranded DNA to the target region is inhibited.

In the latter instance, the probe may have a sequence such that when hybridised with the strand the spaced apart upstream and downstream regions of the strand are drawn toward each other forming a loop or bubble which incorporates the target region. The probe may for instance have opposite end regions which hybridise with the strand and a middle region of non-complementary sequence that does not hybridise with the target region of the strand such that a loop or bubble incorporating the target region is formed and hybridisation of the other strand of the double stranded DNA with the target region is thereby inhibited. To facilitate the formation of the loop or bubble, the middle region of the probe may incorporate inverted repeats that hybridise together following hybridisation of the probe with the strand.

To detect the presence or level of alkylated cytosine in the target region of the single stranded DNA reacted with the enzyme, the target region will typically be amplified and the resulting amplicon(s) analysed for sequence modifications arising from the enzymatic modification of the target region by the enzyme. Hence, a method of the invention may further comprise:
amplifying the target region of the single stranded DNA reacted with the enzyme utilising a process involving thermocycling and primers to obtain an amplified product; and
analysing the amplified product for sequence variations consistent with the presence of alkylated cytosine in the target region of the single stranded DNA.

Determination of the level of alkylated cytosine may be achieved using any technique capable of detecting sequence modifications such as point mutations. Such techniques include, but are not limited to, nucleic acid sequencing and polymerase chain reaction (PCR) techniques, restriction enzyme digests, and techniques involving the use of probes that bind to specific nucleic acid sequences. The determination may comprise quantitative and/or qualitative analysis of the alkylated cytosine content of the target region of the single stranded DNA. In particular, hypermethylation or hypomethylation may be detected by a method of the invention and more particularly, patterns of cytosine alkylation in the DNA.

The DNA evaluated may comprise a gene or a region thereof and preferably, a regulatory non-coding region of a gene such as a 5' non-coding region. The 5' non-coding region may comprise the promoter or promoter region of a gene. Typically, the double stranded DNA will be genomic DNA.

Accordingly, in another aspect of the present invention there is provided a method for detecting the presence or level of alkylated cytosine in a sample of genomic DNA from an individual, the method comprising:
(a) converting at least one region of the genomic DNA to single stranded DNA;
(b) reacting a target region of the single stranded DNA from step (a) with at least one enzyme having cytidine deaminase activity, the enzyme differentially modifying alkylated cytosine and cytosine; and
(c) determining the level of enzymatic modification of the target region by the enzyme.

In a still further aspect of the present invention there is provided a method involving detecting the presence or level of alkylated cytosine in a sample of genomic DNA from an individual, the method comprising:
(a) converting at least one region of the genomic DNA to single stranded DNA;
(b) reacting a target region of the single stranded DNA from step (a) with at least one enzyme having cytidine deaminase activity, the enzyme differentially modifying alkylated cytosine and cytosine; and
(c) determining the level of enzymatic modification of the target region by the enzyme;
for use in the diagnosis of a disease or condition in an individual.

Typically, the enzyme used in a method of the invention will be a deaminase enzyme. The alkylated cytosine detected will generally be 5-alkylcytosine and usually, 5-methylcytosine. The presence of 5-methylcytosine is a useful marker in many conditions and disease states, and for upregulated or downregulated gene expression. Detection of the presence of 5-methylcytosine is also useful in mutation and epigenetic polymorphism analysis.

Accordingly, the detection of 5-methylcytosine in DNA has significant diagnostic and other applications.

The term "individual" as used herein is to be taken in the broadest sense and is intended to include within its scope human beings and non-human animals, bacteria, yeast, fungi and viruses.

Any discussion of documents, acts, materials, devices, articles or the like which has been included in the present specification is solely for the purpose of providing a context for the present invention. It is not to be taken as an admission that any or all of these matters form part of the prior art base or where common general knowledge in the field relevant to the present invention as it existed anywhere before the priority date of each claim of the application.

Throughout this specification the word "comprise", or variations such as "comprises" or "comprising", will be understood to imply the inclusion of a stated element, integer or step, or group of elements, integers or steps, but not the exclusion of any other element, integer, or step, or group of elements, integers or steps.

The features and advantages of methods falling within the scope of the present invention will become further apparent from the following description of preferred embodiments of the invention.

### BRIEF DESCRIPTION OF THE ACCOMPANYING DRAWINGS

Figure 1: Illustrates methodology for creating synthetic internal control DNA for use in an embodiment of a method of the invention.
Figures 2A-2C: Illustrate a primer extension assay for enzyme mediated deamination in *E-cadherin* DNA.
Figure 3A: Illustrates a scheme for using DNA oligonucleotides (looping probes) which are complementary to the target sequence for "looping out" regions of the target region of a gene.
Figure 3B: Illustrates a scheme for using DNA oligonucleotides (antisense probes) which are complementary to the non-target sequence to loop out regions of the target gene by binding to the complementary strand of DNA.
Figure 3C: Illustrates the choice of restriction enzymes to provide selective digestion with Exonuclease III of the non-target strand of the *E-cadherin* promoter sequence surrounding position #972 (GenBank Accession # L34545).

### DETAILED DESCRIPTION OF THE INVENTION

Generally, the enzyme used in a method at the present invention will have cytidine or cytosine deaminase activity, and be able to deaminate cytosine bases in genomic DNA to uracil without substantially deaminating any 5-methylcytosine bases in the DNA. The enzyme may be a thermostable cytidine or cytosine deaminase derived from a thermophilic organism.

The enzyme may for instance be selected from Activation-Induced Cytidine Deaminase (AID) (GenBank human mRNA Ref. Sequence #NM_020661; Genbank human protein sequence #NP_065712.1), Cytidine Deaminase (also known as Cytidine Aminohydrolase EC 3.5.4.5), Cytosine Deaminase (also known as Cytosine Aminohydrolase EC 3.5.4.1), Deoxycytidine Deaminase (also known as Deoxycytidine Aminohydrolase EC 3.5.4.14), Deoxycytidilate Deaminase (also known as Deoxycytidilate Aminohydrolase), Apolipoprotein B mRNA Editing Enzyme (ApoBRe) and catalytic fragments, homologues and variants thereof. By catalytic fragment is meant an enzyme fragment possessing some or all of the catalytic activity of the complete enzyme. Generally, a catalytic fragment utilised in a method of the invention will have substantially the same catalytic activity as the complete enzyme. Catalytic fragments of ApoBRe include APOBEC1 (Catalytic Polypeptide 1, transcript variant 1: GenBank human mRNA Ref. Sequence #NM_001644, Genbank human protein sequence #NP_001635.1; Catalytic Polypeptide 1, transcript variant 2: GenBank human mRNA Ref. Sequence #NM_005889, Genbank human protein sequence #NP_005880.1). Homologues of APOBEC1 include APOBEC2 and APOBEC3A to APOBEC3G, and one or more of such homologues may also be utilised in a method described herein. Sequence data for these homologues is also publicly available from the GenBank database, National Center for Biotechnology Information, Rockville Pike, Bethesda, MD, USA (APOBEC2: GenBank human mRNA Ref. Sequence #NM_006789, Genbank human protein sequence #NP_006780.1; APOBEC3A: GenBank human mRNA Ref. Sequence #NM_145699, Genbank human protein sequence #NP_663745.1; APOBEC3B: GenBank human mRNA Ref. Sequence #NM_004900, Genbank human protein sequence #NP_004891.3; APOBEC3C: GenBank human mRNA Ref. Sequence #NM_014508, Genbank human protein sequence #NP_055323.2; APOBEC3D: GenBank human mRNA Ref. Sequence #NM-152426, Genbank human protein sequence #NP_689639.1; APOBEC3E: GenBank human mRNA Ref. Sequence #NG-002331; APOBEC3F: GenBank human mRNA Ref. Sequence #NM_145298, Genbank human protein sequence #NP_660341.2; APOBEC3G: GenBank human mRNA Ref. Sequence #NM_021822, Genbank human protein sequence #NP_068594.1).

The enzyme utilised may also be a mutant form of such wild type enzymes or catalytic fragments or homologues, which has cytidine or cytosine deaminase activity. Such mutant enzymes may be isolated from nature or generated by rational or random mutation protocols known in the art (e.g. see Twyman R.M. Recombinant DNA and molecular cloning. Chapter 24. In: Advanced Molecular Biology: A Concise Reference. BIOS Scientific Publishers Limited (39)). All such mutant enzyme forms having the desired activity may be employed in methods of the invention. Moreover, a single enzyme may be utilised in a method described herein or combinations of different enzymes with the desired activity independently selected from for instance wild-type enzymes, and variants, homologues, modified and mutant forms, and catalytic fragments thereof.

Enzymes with cytosine deamination activity can be purified from a number of sources including B-cell lymphocytes and transduced expression systems, such as E-coli and insect cells. AID for instance may be expressed as a GST fusion protein in a baculovirus system in insect cells and affinity column purified (Bransteitter 2003. PNAS 100:4102).

Genomic DNA will usually be utilised in a method of the invention and may be extracted from any cells or biological samples deemed appropriate. Genomic DNA extracted by standard protocols is fragmented to varying degrees and is largely double stranded. Activation-Induced Cytidine Deaminase, and other enzymes with cytidine deaminase activity, typically have highest activity on single stranded DNA, or on regions of single stranded loops in double stranded DNA (27). Double stranded DNA can be made single-stranded by a variety of methods including heat denaturation, chemical denaturation, protein binding and exonuclease activity and any of these techniques may be utilised.

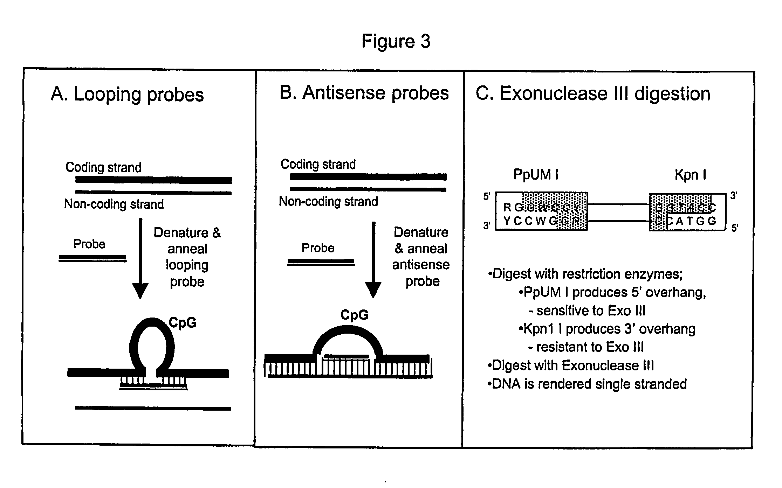

Heat denaturation is commonly used for generating single-stranded DNA and is used in processes such as PCR. Chemical denaturation involves incubation in chemicals such as alkali (7, 32) or formamide (32). Incubation with proteins that bind single-stranded DNA such as Bacteriopharge T4 gene 32 protein (and truncated forms of this protein) destabilise the double helix of genomic DNA and reduces secondary structures (33, 34). Enzymatic denaturation can also be used involving selective enzymatic degradation of one strand of double stranded DNA by incubation with exonucleases such as Exonuclease III from *E. coli* which catalyses the 3'to 5' removal of mononucleotides from 3'-hydroxy termini of duplex DNA. Exonulease III has been used to prepare single-stranded DNA substrates (see Figures 3A-3C) for dideoxy sequencing (35), direct sequencing using MALDI-TOF mass spectroscopy (36) and single-strand conformation polymorphism analysis (32).

Nucleotide analogues, such as Peptide Nucleic Acids (PNA) and Locked Nucleic Acids (LNA), bind to both RNA and DNA with high sequence specificity and affinity. The analogue DNA duplex is more stable than DNA:DNA bonds and oligonucleotide probes containing nucleotide analogues can demonstrate strand invasion properties. For example, PNA probes have the ability to invade double stranded DNA through the generation of a stable PNA₂:DNA triplex (composed of both Hoogsteen and Watson/ Crick base pairing) and strand displacement. PNA probes demonstrate utility both *in-vitro* (single nucleotide polymorphism detection (40)) and *in-vivo* (blocking access to enzymes such as T7 RNA polymerases, transcriptional activation factors, nucleases, restriction enzymes and methylases (41)). Accordingly, strand displacing probes, complementary to the antisense strand of interest (ie. the same sequence as the target sense strand) may render the target sense strand single stranded and therefore available as a target for cytosine deaminase activity.

Strand displacement probes can be designed to bind via duplex, triplex invasion or non-invasive triplex formations (42, 43) and their use can render the prior-DNA denaturation step redundant. The binding kinetics and specificity of strand displacement probes may be improved / altered by reaction conditions and modifications to their design. The design of strand displacing probes can include mono-PNAs, bis-PNAs (which form P-loops when bound to dsDNA), bis-PNA openers (44), the addition of cationic residues such as lysine and the incorporation of pseudoisocytosine (J-bases) (45). Hence, the invention expressly extends to the use of nucleic acid analogue probes comprising or consisting of nucleotide analogues such as PNA and LNA for at least partially separating double stranded DNA.

Probes utilised for hybridising with the single stranded DNA generated by separation of the strands of the genomic DNA to inhibit the annealing of the separated strands and thereby allowing access of the enzyme to the target region of interest, will generally be synthetic oligonucleotide probes or nucleic acid analogue probes. More particularly, the or each probe may be independently a DNA probe or an analogue thereof such as an RNA, PNA, or LNA probe, or other nucleic acid analogue probe comprising one or more nucleotide analogues comprising or consisting of nucleotide analogues. Moreover, the probe(s) may be selected from sense probes, antisense probes, looping probes, and combinations thereof. Typically the probes will be incapable of acting as primers and being extended during PCR. The probes will generally be about 10 bases in length, usually between about 10 and 50 bases in length and preferably, be about 17 to about 30 bases in length. However, longer probes are not excluded and may be used for generating a plurality of loops or bubbles along the length of the DNA strand to be assayed for facilitating reaction of the enzyme with multiple sites along the strand (see Figures 3A-3C).

Nucleotide analogues which may find use in probes include, but are not limited to, the analogues in the following list.

| **Abbreviation** | **Name** |
|---|---|
| ac4c | 4-acetylcytidine |
| chm5u | 5-(carboxyhydroxylmethyl)uridine |
| Cm | 2'-O-methylcytidine |
| cmnm5s2u | 5-carboxymethylaminomethyl thiouridine |
| D | dihydrouridine |
| Fm | 2'-O-methylpseudouridine |
| Galq | β, D-galactosylqueosine |
| gm | 2'-O-methylguanosine |
| I | Inosine |
| i6a | N6-isopentenyladenosine |
| mla | 1-methyladenosine |
| mlf | 1-methylpseudouridine |
| mlg | 1-methy(guanosine |
| ml1 | 1-methylinosine |
| m22g | 2,2-dimethylguanosine |
| m2a | 2-methyladenosine |
| m2g | 2-methylguanosine |
| m3c | 3-methylcytidine |
| m5c | 5-methylcytidine |
| m6a | N6-methyladenosine |
| m7g | 7-methylguanosine |
| mam5u | 5-methylaminomethyluridine |
| mam5s2u | 5-methoxyaminomethyl-2-thiouridine |
| manq | β, D-mannosylmethyluridine |
| mcm5s2u | 5-methoxycarbonylmethyluridine |
| mo5u | 5-methoxyuridine |
| ms2i6a | 2-methylthio-N6-isopentenyladenosine |
| ms2t6a | N-((9-β-ribofuranosyl-2-methylthiopurine-6-yl)carbamoyl)threonine |
| mt6a | N-((9-β-ribofuranosylpurine-6-yl)N-methyl-carbamoyl)threonine |
| mv | Uridine-5-oxyacetic acid methylester |
| o5u | Uridine-5-oxyacetic acid (v) |
| osyw | Wybutoxosine |
| p | Pseudouridine |
| q | Queosine |
| s2c | 2-thiocytidine |
| s2t | 5-methyl-2-thiouridine |
| s2u | 2-thiouridine |
| s4u | 4-thiouridine |
| t | 5-methyluridine |
| t6a | N-((9-β-D-ribofuranosylpurine-6-yl)carbamoyl)threoninetm 2'-O-methyl-5-methyluridine |
| um | 2'-O-methyluridine |
| yw | Wybutosine |
| x | 3-(3-amino-3-carboxypropyl)uridine, (acp3)u |
| araU | β, D-arabinosyl |
| araT | β, D-arabinosyl |

Incubation of the single stranded genomic DNA with an enzyme with a preferential ability to deaminate cytosine and not 5-methylcytosine results in a sequence with uracil in place of cytosine residues but with 5-methylcytosine residues substantially unchanged. The optimum reaction conditions for reaction of the DNA with the selected deaminase enzyme may be determined by altering one or more reaction conditions utilised.

Genomic DNA from the colorectal cancer cell line SW480 shows complete 5-methylation of the cytosines present in CpG sites in the CpG island in the promoter region of the *p16* gene. The DNA in this region of the gene also contains unmethylated cytosines. Genomic DNA from this cell line therefore provides a model substrate on which to test reaction variables to determine the optimum conditions for maximum discrimination between cytosine and 5-methylcytosine incorporated into DNA as substrates for deamination by enzymes with cytidine deaminase activity.

For determining optimum reaction conditions, genomic DNA can be extracted from the SW480 cells using standard methods. The DNA from the cells is then converted to single stranded DNA, preferably by heat denaturation. The re-annealing of the separated strands can be inhibited using probes as described above. The promotor region of the *E-cadherin* gene, which contains three CpG islands with multiple CpG sites can also be used to optimise reaction conditions or for assessing enzymes such as AID and other deaminase enzymes for use in a method of the invention.

The capacity of an enzyme to differentially modify alkylated cytosine and cytosine can be tested by adding it to the single stranded DNA and incubating under a range of variables selected from for instance; the concentration of DNA, the concentration of enzyme, the time of incubation, the temperature of incubation, the composition and concentration of the buffering ion (commonly used buffers include TRIS, HEPES, MOPS & imidazole), the pH of the buffer (from pH 4.0 to 10.0), the concentration and type of salt (commonly used salts include sodium chloride, sodium acetate, potassium chloride, potassium acetate, salts of sulphate and salts of ammonium), the concentration of various cationic metal ions (for example magnesium, manganese, lead, and calcium), the concentration of various protein stabilisers if any (for example reducing agents such as dithiothreitol (DTT), other proteins (such as bovine serum albumin (BSA)), sugars (such as sucrose, maltose, glucose, trehalose, glycerol and fructose), detergents (such as Triton®X-100 and Tween-20), and co-solvents (such as proline, betaine, formamide, DMSO, alcohols and polyols). The degree of discrimination between cytosine and 5-methylcytosine achieved using different combinations of these variables can then be assessed by protocols as further described below. Those skilled in the art will appreciate that the above list of reaction condition variables is not exclusive and further examples of reagents and conditions that alter or enhance substrate specificity and rates of reaction of enzymes can be found in publicly available literature.

Besides PCR product and genomic DNA, chemically synthesized oligonucleotides can be utilised for determining optimum reaction conditions for the selected enzyme, and may be produced with and without 5-MeC bases. Such chemically synthesized oligonucleotides can provide substrates for the enzyme as single stranded DNA or double stranded DNA (annealed to a chemically synthesized antisense strand). The latter are useful for optimising methods and reaction conditions to render double stranded substrates single stranded for maximising accessibility to deaminase enzymes such as AID which require single stranded substrate for greatest activity. Moreover, cytosine nucleotides in PCR product can be methylated by incubation in methyltransferases such as MsssI, which recognizes and methylates cytosine in the sequence 5'..CG .. 3', and HpaII, which methylates the internal cytosine in 5'.. CCGG .. 3' sequences. Genomic DNA from cell lines or normal human donors can also serve as substrates for optimising enzyme-mediated cytosine deamination. The methylation status of cytosine nucleotides in any of the above substrates (chemically synthesized oligonucleotides, PCR product or genomic DNA) can be assessed by standard conventional bisulfite modification and sequencing methods.

Following incubation of the test DNA with the enzyme, the target region of interest in the DNA will typically be amplified by PCR. Generally, the enzyme will be heat denatured before the commencement of the PCR. Using the modified DNA as a template in PCR results in an amplified sequence (amplicon) with thymidine residues in place of cytosine in the original sequence and cytosine in place of 5-methylcytosine. Accordingly, the conversion of cytosine bases to uracil by the enzyme, followed by conversion to thymine by the PCR, creates a modified DNA with sequence differences associated with the methylation status of the cytosines in the original DNA template.

These sequence variations can be detected using any protocol which can discriminate between thymidine and cytosine bases, including techniques such as direct sequencing of the region (e.g. see Herman *et al* (10)), digestion of the PCR amplicon with restriction enzymes, methylation-specific PCR (10), Restriction Endonuclease Mediated Selective PCR (REMS-PCR) (eg.(37); International Patent Application No. PCT / AU96 / 00213 published as WO 9632500) and hybridisation with methylation-specific probes (11). Methylation-specific PCR relies on primers that take advantage of the sequence differences between methylated and non-methylated regions after conversion by the enzyme. All of these methods will give information on the methylation status of cytosines in the target region of the test DNA being assayed.

The selective nature of amplification by REMS-PCR means that it is well suited for analysis of rare genetic variations such as tumour sequences in a background of normal sequences, or foetal sequences in a background of maternal sequences. Accordingly, a method of the invention may form the basis of minimally invasive assays in which body fluids are analysed for the presence of variant sequences characterised by altered or aberrant cytosine methylation patterns.

The method of the present invention may be used to detect hypermethylated sequences within the promoter region of genes in association with human tumours such as for example, hypermethylation in the CpG island within the *p16* gene promoter. Hypermethylation of this region has been detected in bladder, breast, gastric, head and neck, oesophageal, colon, lung and liver cancer as described above. Other examples of genes which have CpG island hypermethylation in association with human tumours include *E-cadherin* (breast, prostate, colon, bladder, and liver tumours), the von Hippel Lindau (VHL) gene (renal cell tumours), *BRCA1* (breast tumours), *p15*(leukemias, Burkitt lymphomas), hMLH1 (colon tumours), ER (breast, colon, lung tumours, and leukemias), HIC1 (brain, breast, colon, and renal tumours), MDG1 (breast tumours), GST-π (prostate tumours), O⁶-MGMT (brain tumours), calcitonin (carcinoma and leukemia), and *myo-D* (bladder tumours) (1, 3).

A method as described herein can also be used to identify regions of hypomethylation, such as regions of hypomethylation associated with the transcriptional activation of genes, for example, urokinase or S100A4 in cancer.

Accordingly, altered methylation patterns may be used as markers of tumour cells. Specific applications utilising such markers include for example, minimally invasive screening or early diagnosis of tumours or cancers, detection of micrometastatic or metastatic disease in lymph nodes, detection of unresected tumour cells at tumour margins or other residual disease, or as a tool for predicting relapse. In addition, differences in patterns of 5-methylcytosine bases at discreet genetic loci may be used as a marker for foetal DNA or disease states such as fragile X syndrome and altered gene imprinting states. The presence of 5-methylcytosine may also provide a marker of endogenous or exogenous DNA associated with viruses, bacteria or other such microorganisms or pathogens, and so provide a means for indicating infection by the pathogen, or microorganisms, or of identifying the pathogen or microorganism.

Optimisation of buffer, ionic strength, pH and other reaction conditions for rendering DNA single stranded and combinations of difference enzymes, may allow essentially total deamination of the target bases in the DNA to be reached. However, total deamination is not an absolute requirement for methylation analysis. For example, the presence of methylated cytosine can be detected by comparison between the rate (extent) of deamination at a target site against an internal control. The internal control can be a site within genomic DNA that is known to be unmethylated, or it may be synthetic unmethylated DNA that is spiked into the reaction. Quantification of the rate of deamination at the two target sites (target and internal control) may for instance be achieved using real time quantitative methylation specific PCR (MSP) (11, 46) protocols, by comparison of the percentage cleavage in COBRA assays (4), or by comparison of band intensities on sequencing gels (8).

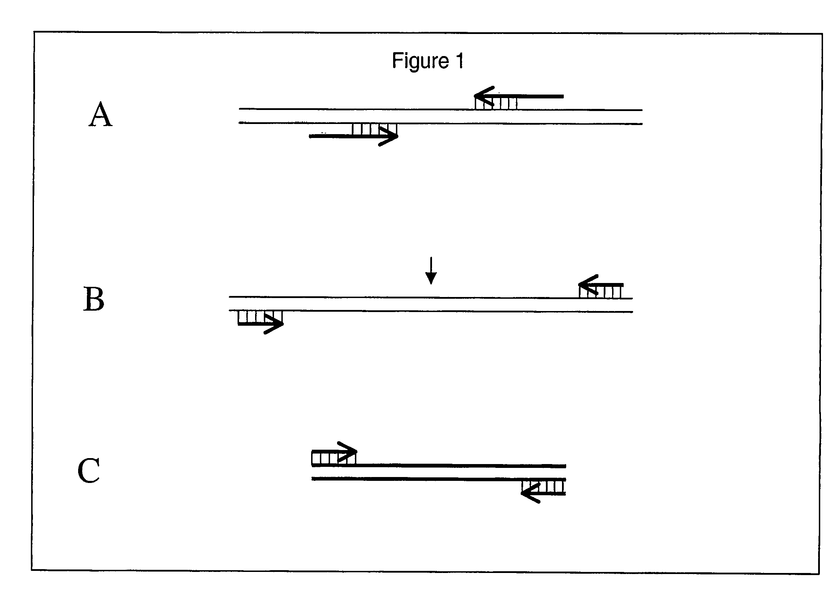

One method that may be used to create a synthetic internal control is illustrated in Figure 1. More particularly, to prepare the internal control, an internal fragment of genomic DNA template is amplified with primers that have 3' termini which are complementary with the genomic DNA and non-complementary 5' tags (A). The genomic DNA is then amplified using outer primers that are specific for the genomic DNA and which will not amplify the internal control fragment (B). Similarly, the internal control fragment is amplified using primers that are specific for the internal control fragment and do not amplify the genomic DNA (C).

Alternatively, controls can be in separate reactions. For instance, genomic DNA may be analysed employing quantitative real time MSP as described above, and three standards curves constructed using bisulfite treated methylated genomic DNA (M standards), bisulfite treated unmethylated genomic DNA (U standards) and untreated unmethylated genomic DNA (W standards). A methylation index (%MI) can then be calculated as %MI=M ÷ (M + U) x 100. The %MI calculated does not take into account the percentage of DNA (%W) which is not converted from C to U by bisulfite treatment (the background) calculated as %W = W ÷ (W + M + U) x 100. Each of the values M, U and W are estimated for the test DNA sample with reference to the respective standard curves (46). To remove the background from the % MI the following calculation can be employed: %MI (minus background) = %MI x (1 - slow ÷ 100)). This formula, therefore, allows an estimate of the true amount of methylated cytosine in the genomic DNA test sequence analysed where conversion of cytosine to uracil was less than 100%.

Control DNA sequence of known cytosine methylation status for optimising reaction conditions or assessing the efficacy of the enzymatic modification includes controls include plasmids, PCR fragments generated by replacing dCTP with methy⁵-dCTP (38), and commercially available human genomic that is DNA universally methylated for all genes (CpGenome^{™} Universally Methylated DNA, Intergen Company, Cat. No. S7821). In addition, cell line DNA, extracted from cell lines with a known methylation status may be used for positive and negative controls. As an example, the CpG dinucleotides in the CpG island in the promoter region of the *p16*gene are fully methylated in the lung cancer cell lines H157 and U1752, and unmethylated in the lung cancer cell lines H249 and H209 (10). The genomic DNA maybe extracted from the cell lines by standard protocols known in the art.

Enzymatic modification of cytosine bases in the test DNA being assayed will generally be carried out using the minimum incubation period deemed necessary to achieve modification of the cytosine bases in the DNA by the enzyme utilised, and in conditions that do not lead to excessive fragmentation of the DNA. Advantageously, the protocol will typically be faster than conventional DNA modification protocols known in the art.

In order that the nature of the present invention may be more clearly understood, preferred forms thereof will now be described with reference to the following non-limiting examples.

### Example 1: Enzymatic conversion of genomic DNA using Activation-Induced Cytidine Deaminase for the detection of the methylation status of the CpG Island in the promoter of the p16 (INK4a) gene.

Genomic DNA is first extracted from a blood or tissue sample from the individual using a standard extraction protocol known in the art. Human genomic DNA, universally methylated for all genes (CpGenome^{™} Universally Methylated DNA), is used as a positive control for detection of 5-methylcytosine within the CpG island in the promoter of the *p16*gene.

Single stranded DNA is generated from the double stranded genomic DNA by heat denaturation. The resulting single-stranded DNA is subsequently incubated with Activation-Induced Cytidine Deaminase in conditions that promote deamination of cytosine bases in the DNA, but not 5-methylcytosine bases. Activation-Induced Cytidine Deaminase can be prepared in a number of ways including as a crude extract from activated B-cells (28), and expression of a fusion protein to facilitate purification (26, 27).

The area of interest around the CpG island of the *p16*promoter (GenBank Accession No. X94154) is then amplified by PCR. Primers are chosen in regions that are not methylation hot-spots to reduce the possibility of efficiency of amplification being dependent on methylation status. Suitable primer sequences are described in Herman *et al.* (10). The PCR product contains thymidine bases where unmethylated cytosine existed in the template genomic DNA and cytosine bases where 5-methylcytosine bases existed in the template genomic DNA. The methylation status of the CpG island in the promoter region of the *p16*gene is then assessed using a suitable protocol as described above by comparison to known reference sequence. Detection of methylated CpG sequences within the CpG island in the promoter region of *p16*may be used as a marker of tumours of several organs including the bladder, breast, gastric, head and neck, oesophageal, colon, lung or liver.

### Example 2: Enzymatic conversion of genomic DNA using Activation-Induced Cytidine Deaminase to facilitate detection of the methylation status of the individual CpG dinucleotides in the CpG island in the promoter of the p16(INK4a) gene.

As in Example 1, genomic DNA is first extracted from a blood or tissue sample from the individual using a standard extraction protocol known in the art. Human genomic DNA, universally methylated for all genes (CpGenome^{™} Universally Methylated DNA), is used as a positive control for detection of 5-methylcytosine within the CpG island in the promoter of the *p16*gene (also called the CDKN2 gene, GenBank Accession No. X94154).

Specific areas of the CpG island in the promoter of the *p16* gene are targeted for enzymatic conversion by Activation Induced Cytidine Deaminase by using a synthetic DNA probe with areas of complementarity around the CpG sequence to be analysed such that hybridization of the DNA probe produces a central loop of single stranded DNA containing the CpG sequence, or sequences, to be analysed. The DNA probe is hybridized to the genomic DNA by mixing the probe and the genomic DNA together, then heat denaturing the genomic DNA and cooling the solution to a temperature lower than the melting-temperature of the probe. In a variation of this technique, a plurality of such DNA probes may be hybridised with the genomic DNA to target a number of regions of interest in the genomic DNA. In a further variation of this technique, the probes may contain modified DNA bases such as PNA or LNA.

The genomic DNA with the DNA probe hybridised to it is subsequently incubated with Activation-Induced Cytidine Deaminase under conditions that promote deamination of cytosine bases in the genomic DNA by the enzyme, but not 5-methylcytosine bases.

The area of interest around the CpG island of the *p16*promoter is then amplified by PCR. The PCR product will contain thymidine bases where unmethylated cytosine existed in the loop of template genomic DNA, and cytosine bases where 5-methylcytosine bases existed in the template genomic DNA. The methylation status of the CpG island in the promoter region of *p16* is then assessed as in Example 1.

Methylation-specific PCR relies on primers that take advantage of the sequence differences between methylated and unmethylated regions after conversion by an agent such as bisulfite. To detect the CpG dinucleotides targeted for enzymatic conversion by Activation Induced Cytidine Deaminase using methylation specific PCR, methylation-specific primers are designed to this region.

### Example 3: AID-mediated cytosine deamination of single stranded DNA

### A. Preparation of substrate

An unmethylated 80bp oligonucleotide (Ecad80) which has the same nucleotide sequence as nucleotide bases #920 to #999 of the *E-cadherin* promoter region (GenBank Accession #L34545), was diluted to 4 µM in 50 mM NaCl. The sequence of Ecad80 is as follows: 5' cgc tgc tga ttg gct gtg gcc ggc agg tga acc ctc agc caa tca gcg gta Cgg ggg gcg gtg ctc cgg ggc tca cct gg 3' SEQ ID NO:1. Nucleotide base #52 in this sequence (upper case C) was screened with the primer 3ECAD11b in the cycle sequencing primer extension assay described below in D, and corresponds to base #972 of *E-cadherin* promoter region (GenBank Accession # L34545).

### B. Trap DNA annealing

Complementary oligonucleotides AA1 (tgt ttt ggg tgt gta tgg ttt ggg tgt) SEQ ID NO:2 and AA2 (aca ccc aaa cca tac aca ccc aaa aca) SEQ ID NO: 3 were diluted to 30 µM each in 20 mM NaCl and the mixture was heated to 95°C for 5 min, and cooled slowly to room temperature to allow annealing of the complementary strands. The resulting double stranded "TRAP DNA" template was used as a decoy for exonuclease activity in the following 20 µL AID reaction mixture.

### C. AID mediated cytidine deamination reactions

The 20 µL AID reaction mixture contained 50 mM Hepes pH 7.5, 1 mM DTT, 10mM MgCl₂, 24 pmole Trap DNA (AA1/AA2), 4 pmole Ecad80 substrate, 200 ng RNase A, and 100 nM wildtype AID. The enzyme mixture was incubated at 37°C and stopped after 15 minutes by addition of phenol: isoamylalcohol: chloroform (25:24:1, Amresco #0883-100ml). The aqueous phase containing the substrate was separated from the phenol:chloroform phase using Eppendorf Phase Lock Gel™ tubes (Light, 0.5ml Cat. #0032 005.004). The aqueous phase was further purified by eluting the sample through BioRad Micro Bio-spin 6 Chromatography Columns (Cat. #732-6200).

### D. Screening AID-mediated cytosine deamination using cycle sequencing by primer extension

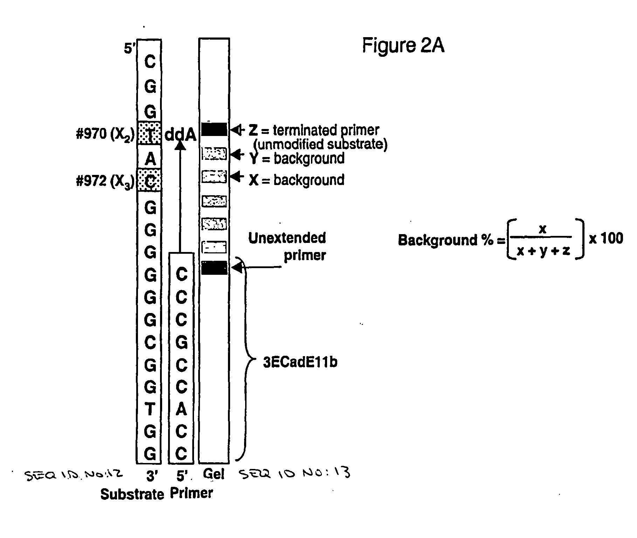

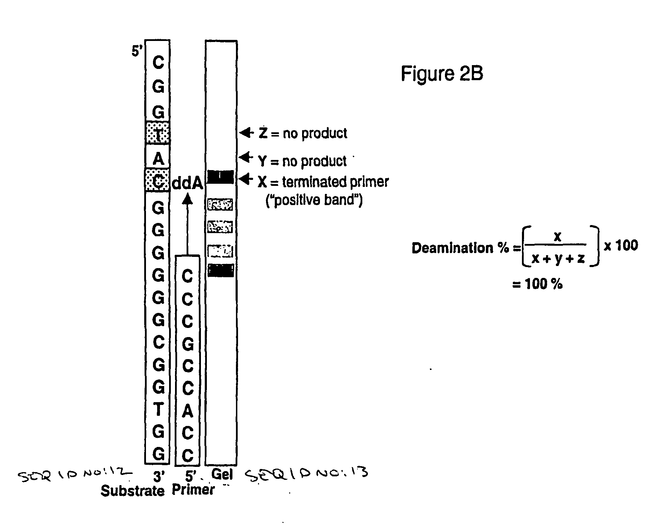

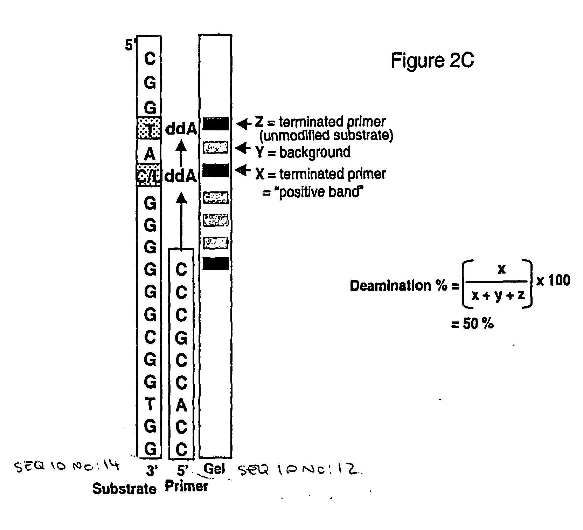

Cycle sequencing primer extension with a ³²P-lablelled primer provides a measure of the degree of cytosine deamination. Incorporation of ddA at a site containing a C in the DNA substrate is consistent with deamination of C to U. This is described in more detail below with reference to Figure 2.

In these reactions, 4 µL of AID modified substrate was amplified using a cycle sequencing protocol. Specifically, cycle sequencing reactions contained 1 x Thermosequenase buffer (USB), 3 Units of Thermosequenase (USB), 67 nM ³²P-end labelled primer 3ECAD11b (5' agc ccc gga gca ccg ccc 3'), 80 µM each of ddATP, dGTP, dCTP and dTTP, and 20 µL mineral oil. The primer 3ECAD11b screens base #972 in the *E-cadherin* promoter region (GenBank Accession # L34545) of genomic DNA or base #52 in Ecad80. Reactions were thermocycled for 7 cycles of (95°C for 30 s, 55°C for 45 s, 72°C for 5 min). Reactions were stopped with 10 µL stop solution containing 95 % formamide, 10 mM EDTA, 0.1% xylene cyanol and 0.1% bromophenol blue, denatured at 95°C for at least 2 minutes and placed immediately on ice. Products were separated on a 20% polyacylamide gel which was run for 3 hours at 60W prior to being dried. Quantitation of the band intensities provides an estimate of the percentage of target template that has been deaminated at position #972.

### E. Interpretation of polyacrylamide results

The resulting banding pattern on the polyacrylamide gel represents the sequence of the template of the cycle sequencing assay. AID-induced deamination of cytosine at position #972 will alter the degree of incorporation of ddA in the primer extension assay with the resulting banding pattern explained as follows. When the reaction contains no AID, the template sequence remains unchanged (Figure 2, Part A). This results in the ³²P-labelled primer extending in the ddA lane until the first T (position #970 in the *E-cadherin* promoter sequence, GenBank Accession # L34545 or position #50 in Ecad80). AID-mediated deamination of the cytosine in position #972 in the *E-cadherin* promoter sequence, or position #52 in Ecad80, to a uracil will result in incorporation of ddA at this site, referred to as a "positive" band (Figure 2, Part B and C). This "positive" band corresponds to a smaller fragment (read through to the first T in the template adds two extra bases to the primer extension product) which runs faster on the polyacrylamide gel. The intensity of the "positive" band can be measured with ImageQuant Software (Molecular Dynamics, USA) and compared with the intensity of all bands above (representing PCR extension beyond this stop point and therefore demonstrating template unconverted at site #972) and including this band. This percentage represents the percentage of cytosine at this position of the substrate which has been deaminated to a uracil by AID.

### F: Discussion

The cycle sequencing reaction indicates that AID mediated deamination of approximately 37% of the cytosine in position #52 of the Ecad80 substrate (measured as described in paragraph E above). The control reaction without AID demonstrates a background level of "positive" band of 4%. This could be a result of either background deamination or misincorporation of ddA by the polymerase. The background level can be taken into account in the assay results by subtracting the control reaction from the test reaction.

**Table 1: AID-mediated cytosine deamination of single-stranded chemically synthesized DNA substrate**

| Reaction | % AID-mediated cytosine deamination |
|---|---|
| Control (minus AID) | 4 |
| Test (plus AID) | 37 |

### Example 4: AID discrimination between unmethylated and methylated cytosine

### A. Preparation of substrate

DNA oligonucleotides were chemically synthesized with the following sequence: cgc tgc tga ttg gct gtg gcX₁ ggc agg tga acc ctc agc caa tca gX₂g gta X₃gg ggg gcg gtg ctc cgg ggc tca cct gg; where X was either unmodified (Ecad80 - all cytosine) SEQ ID NO: 1 or 5'-methylcytosine (5'-MeC) modified (Ecad80M3 - containing three 5-MeC bases at X₁, X₂ and X₃) SEQ ID NO:4. Ecad80 or Ecad80M3 were diluted to 4 uM (in the presence of 50 mM NaCl).

### B: Trap DNA annealing

Complementary oligonucleotides T1 (att ata ttt aaa tat ata aaa tat ata tta ata aat) SEQ ID NO:5 and T2 (att tat taa tat ata ttt tat ata ttt aaa tat aat) SEQ ID NO:6, were diluted to 30µM each in the presence of 20mM NaCl. These oligonucloetides were annealed to function as TRAP DNA as described in Example 3.

### C: AID-mediated cytidine deamination reactions

A 20 µL AID reaction mixture was prepared containing 50 mM Hepes at pH 7.5,1 mM DTT, 10 mM MgCl_{2,} 24 pmole Trap DNA (T1/T2), 4 pmole substrate, 200 ng RNase A, and 100 nM AID. Reactions were incubated at 37°C for 15 minutes.

### D: Cycle sequencing primer extension

The extensions were performed as in Example 3 but using the following thermocycling conditions: 15 cycles of (95°C for 2min, 55°C for 30s, 72°C for 2 min). Polyacrylamide gel was run for 3 hours at 60W and dried for 1 hour before analysis.

### E: Results

The results demonstrate decreased AID-mediated cytosine deamination of methylated cytosine (see Table 2). After 15 minutes reaction time, AID deaminated 35 % of base #52 in Ecad80 compared with only 5 % of base #52 in Ecad80M3.

**Table 2: AID shows less deamination of methylated cytosine than cytosine**

| | |
|---|---|
| Substrate | % AID-mediated cytosine deamination |
| Unmethylated (Ecad80) | 35 |
| Methylated (Ecad80M3) | 5 |

### Example 5: AID-mediated cytosine deamination of genomic DNA

### A: Preparation of genomic DNA as substrate

Genomic DNA was extracted from the human cell line SW480 (#CCL-228) obtained from American Type Tissue Collection (Rockville, Md, USA) using the QIAamp DNA Blood Mini Kit (50) (Qiagen) according to manufacturers directions. Experiments conducted showed genomic DNA from SW480 was unmethylated at #972 of *E-cadherin* promoter region (GenBank Accession #L34545) using standard bisulfite and sequencing methods. The genomic DNA was diluted in sterile water to 10 ng/µL.

### B: AID-mediated cytosine deamination reactions for genomic DNA

All reactions contained 50 mM Hepes at pH 7.5,1 mM DTT, 10 mM MgCl₂, 24 pmole TRAP DNA (AA1/AA2, prepared as in Example 3), 5 ng genomic DNA, 200 ng RNase A and 200 nM AID. Reactions were incubated at 37°C for 15 minutes. Cycle sequencing primer extension and polyacrylamide gel analysis was performed as described in Example 3.

### C: Results

The cycle sequencing results indicate 16% of genomic DNA was converted to uracil by AID-mediated deamination compared with 5% in control reactions without AID.

**Table 3: AID-mediated deamination of genomic DNA**

| Substrate | % AID-mediated cytosine deamination |
|---|---|
| Genomic DNA (minus AID) | 5 |
| Genomic DNA (plus AID) | 16 |

The low level of AID-mediated cytosine deamination on genomic DNA demonstrated here may be due to the presence of low amounts of single stranded DNA in this preparation of genomic DNA or this may be the first demonstration of deamination of cytosine in double-stranded genomic DNA by AID. AID has been shown previously to act on single stranded and not double stranded substrates (27), which explains the low deamination of double stranded genomic DNA.

### Example 6: Enhancing AID-mediated cytosine deamination by use of looping probes and antisense probes to render substrates single stranded

### A: Preparation of substrate

Ecad80 was diluted to 4 µM in 50 mM NaCl with three-fold excess of ASEcad80 which is the antisense sequence of Ecad80 (ASEcad80 sequence: 5' cc agg tga gcc ccg gag cac cgc ccc ccg tac cgc tga ttg gct gag ggt tca cct gcc ggc cac agc caa tca gca gcg 3') SEQ ID NO:7. Mixtures were heated to 95°C for 5 minutes and cooled slowly to room temperature to allow annealing of complementary strands.

### B: Annealing of oligonucleotide looping probes and antisense probes

An excess of oligonucleotide probe, designed to anneal to the target strand and generate single stranded loop at a target site, were added to the double stranded template prepared as in step A of this Example. The following looping probe DNA sequences where chemically synthesized:
LP10 - 5' CGA CCG CCC CGA TTG GCT GAG G 3' (with 3' phosphate); SEQ ID NO:8
LP26 - 5' GCC CCG GAG CGA GGG TTC ACC TG 3' (with 3' phosphate); SEQ ID NO:9; and
LP26+1-5'GCCCCG GAG CGG AGG GTT CAC CTG 3'(with 3' phosphate). SEQ ID NO:10

These looping probes produce single-stranded loops of 10, 26 and 26+1 bases respectively. LP26+1 leaves an unpaired nucleotide on the looping probe (underlined nucleotide) at the opening of the loop to provide increased flexibility. The antisense oligonucleotide AS26 5' AGC CAA TCA GCG GTA CGG GGG GCG GT 3' SEQ ID NO:11 (with 3' phosphate) was chemically synthesized. AS26 anneals with full complementarity to the non-target strand to produce a 26 base loop on the template strand. Mixtures of probes and substrate were heated to 95°C for 5 minutes and allowed to cool slowly to room temperature.

### C: AID-modification of substrate

A 20 µl AID reaction mixture was prepared containing 50 mM Hepes at pH 7.5,1 mM DTT, 10 mM MgCl₂, 24 pmole Trap DNA (AA1/AA2), 4 pmole substrate, 200 ng RNase A, and 100 nM AID. Reactions were incubated at 37°C for 15 minutes.

### D: Cycle sequencing primer extension

Performed as in Example 4. Polyacrylamide gel was run for 3 hours at 60W, prior to being dried for 1 hour 15 minutes and analysed.

### E: Results

Incubation of the double-stranded DNA substrate with oligonucleotide probes designed to create single stranded loops of different sizes (10, 26 or 26+1bp +/- 26bp antisense probe) can increase AID-mediated cytosine deamination as indicated in Table 4.

**Table 4: AID-mediated deamination of double stranded DNA substrate incubated with looping probes and antisense probes**

| | % AID-mediated cytosine deamination | | |
|---|---|---|---|
| Looping probe | LP10 | LP26 | LP26+1 |
| Looping probe alone | 17 | 22 | 22 |
| Looping probe + Antisense probe (AS26) | Not tested | | 29 |

### Example 7: Improving AID-mediated cytosine deamination by changing buffer ion and concentration

The following example was conducted to indicate how reaction conditions may be optimised for a selected enzyme, and the affect of different conditions of buffer ion and concentration on AID-mediated cytosine deamination.

### A: Preparation of substrate

Ecad80 (5' cgc tgc tga ttg gct gtg gcc ggc agg tga acc ctc agc caa tca gcg gta Cgg ggg gcg gtg ctc cgg ggc tca cct gg 3') SEQ ID NO:1 was diluted to 4 µM in 50 mM NaCl.

### B: AID-mediated cytidine deamination of substrate

Reactions contained a range of buffer types and concentration as follows:10, 50 or 100 mM of Tris-HCl, Hepes, Pipes or Imidazole buffering ions. All reactions were conducted at pH 7.5 and contained 1 mM DTT, 10 mM MgCl₂, 24 pmole Trap DNA (T1/T2, prepared as in Example 4), 4 pmol Ecad80, 200 ng RNase A and 100 nM AID. The reactions were incubated at 37°C for 15 minutes. Cycle sequencing primer extension and polyacrylamide gel analysis were performed as described in Example 3.

### C: Cycle sequencing primer extension

The primer 3ECAD11b screens C in Ecad80 as indicated in step A of this Example. This is the equivalent of nucleotide base #972 of the *E-cadherin* promoter region of genomic DNA. The polyacrylamide gel was run for 9 hours at 9 W, before being dried for 1 hour and then analysed.

### D: Results

The species of buffer ion was found to alter the rate of AID-mediated cytosine deamination. Decreasing ionic strength increases AID-mediated cytosines deamination as indicated in Table 5 below. At 50 mM concentration, Tris-HCl promoted higher AID-mediated cytosine deamination compared to imidazole, Pipes or Hepes at the same pH (Table 5). Thus the type of buffering ion, as well as the ionic strength of the buffer, can be tested to find conditions that enhance the cytosine deaminase activity of AID.

**Table 5: Buffering ion and concentration affects AID-mediated cytosine deamination**

| | % AID-mediated cytosine deamination | | | |
|---|---|---|---|---|
| Ion (mM) | Hepes | Pipes | Imidazole | Tris-HCl |
| 100 | 5 | 3 | 25 | 31 |
| 50 | 17 | 9 | 32 | 44 |
| 10 | 45 | 33 | 43 | - |

### Example 8: Enhancement of AID-mediated cytosine deamination by increasing reaction time

### A. Preparation of substrate

The substrate for this study was prepared as in Example 7.

### B. AID-modification of substrate

The AID reaction mixture contained 10 mM Tris-HCl pH 7.5,1 mM DTT,10 mM MgCl_{2,} 24 pmole Trap DNA (T1/T2 sequence, prepared as in Example 4), 4 pmol Ecad80, 200 ng RNase A and 100 nM wildtype AID. The enzyme mixture was incubated at 37°C for 5 or 30 minutes. The cycle sequencing primer extension and polyacrylamide gel analysis was performed as in Example 3 with the exception that the polyacrylamide gel was run for 9 hours at 9 W, and dried for 1 hour before analysis.

### C: Results

Increasing the incubation time of the AID reactions was found to increase the amount of AID-mediated cytosine deamination as indicated in Table 6. For example, AID-mediated cytosine deamination of Ecad80 was almost doubled from 24 to 44 % by extending the incubation time from 5 to 30 minutes.

**Table 6: Effect of reaction time on AID-mediated cytosine deamination**

| Reaction time (min) | % AID-mediated cytosine deamination |
|---|---|
| 5 | 24 |
| 30 | 43 |

### Example 9: Enhancing AID-mediated cytosine deamination by increasing the amount of AID in reactions

### A: Preparation of substrate

The substrate for this study was prepared as in Example 7.

### B: AID-modification of substrate

The AID reaction mixture contained 10 mM Tris-HCl pH 7.5,1 mM DTT,10 mM MgCl₂, 24 pmole Trap DNA (T1/T2 sequence, prepared as in Example 4), 4 pmol Ecad80, 200 ng RNase A and 100 nM or 200 nM AID. The enzyme mixture was incubated at 37°C for 20 or 30 minutes. The cycle sequencing primer extension and polyacrylamide gel analysis was performed as in Example 3 with the exception that the polyacrylamide gel was run for 9 hours at 9 W, and dried for 1 hour prior to analysis.

### C. Results

Increasing the concentration of AID was found to increase the amount of AID-mediated cytosine deamination as indicated in Table 7.

**Table 7: Effects of increasing the concentration of AID on AID-mediated cytosine deamination**

| | % AID-mediated cytosine deamination | |
|---|---|---|
| Reaction time (min) | 100nM AID | 200nM AID |
| 20 | 34 | 43 |
| 30 | 44 | 54 |

### Example 10: Enzyme-mediated cytosine deamination with APOBEC3G, wildtype and AID mutant R35E/R36D

Cytosine deaminase activity can be produced by native enzymes including AID and the APOBEC homologues. Mutant versions of these proteins can also be produced, either by random or rational mutation, and the mutants screened to find proteins with greater rates of deamination and discrimination between cytosine and 5-methylcytosine.

### A: Preparation of substrate

The substrate for this study was prepared as in Example 7.

### B: Enzyme-modification of substrate

The AID reaction mixture contained 10 mM Tris-HCl pH 7.5,1 mM DTT, 10 mM MgCl₂, 24 pmole Trap DNA (T1/T2 sequence, prepared as in Example 2), 4 pmol substrate, 500 ng RNase A and either 100 nM wildtype AID, 100 nM APOBEC3G or 100 nM of AID mutant R35E/R36D (provided by Prof. Myron Goodman, Dept. of Molecular Biology and Chemistry, University of Southern California, USA). Reactions were incubated at 37°C for 15 minutes.

### C: Cycle sequencing primer extension and polyacrylamide gel analysis

Cycle sequencing primer extension was performed as in Example 4 with the following thermocycling protocol: 20 cycles of (95°C for 2 min, 55°C for 30s and 72°C for 2 min). The polyacrylamide gel was run for 9 hours at 9 W, before being dried for 1 hour and analysed.

### D. Results

The results shown in Table 8 demonstrate that the AID mutant R35E/R36D has higher activity than wildtype AID in deaminating nucleotides base #52 of Ecad80. AID mutant R35E/R36D deaminated almost twice as much substrate as the wildtype protein. APOBEC3G showed a lower amount of cytosine deamination activity but further optimization of reaction conditions (eg buffer species, buffer concentration, pH and enzyme concentration) may improve the rate of APOBEC3G-mediated cytosine deamination. This example further demonstrates the utility of assessing mutants and natural variants of enzymes with cytosine deaminase activity.

**Table 8: Enzyme mediated cytosine deamination**

| | % AID-mediated cytosine deamination |
|---|---|
| Wildtype AID | 32 |
| AID mutant R35E/R36D | 60 |
| APOBEC3G | 22 |

The present embodiments described are to be considered in all respects as illustrative and not restrictive.

### REFERENCES CITED:

1. Baylin, S. B., Herman, J. G., Graff, J. R., Vertino, P. M., and Issa, J. P. Alterations in DNA methylation: a fundamental aspect of neoplasia. Adv Cancer Res, 72:141-196, 1998.
2. Rein, T., DePamphilis, M. L., and Zorbas, H. Identifying 5-methylcytosine and related modifications in DNA genomes. Nucleic Acids Res, 26:2255-2264,1998.
3. Laird, P. W. The power and the promise of DNA methylation markers. Nat Rev Cancer, 3: 253-266., 2003.
4. Xiong, Z. and Laird, P. W. COBRA: a sensitive and quantitative DNA methylation assay. Nucleic Acids Res, 25:2532-2534., 1997.
5. Nakamura, N. and Takenaga, K. Hypomethylation of the metastasis-associated S100A4 gene correlates with gene activation in human colon adenocarcinoma cell lines. Clin Exp Metastasis, 16: 471-479, 1998.
6. Fritzsche, E., Hayatsu, H., Igloi, G. L., Iida, S., and Kossel, H. The use of permanganate as a sequencing reagent for identification of 5- methylcytosine residues in DNA. Nucleic Acids Res, 15: 5517-5528, 1987.
7. Frommer, M., McDonald, L. E., Millar, D. S., Collis, C. M., Watt, F., Grigg, G. W., Molloy, P. L., and Paul, C. L. A genomic sequencing protocol that yields a positive display of 5- methylcytosine residues in individual DNA strands. Proc Natl Acad Sci U S A, 89:1827-1831, 1992.
8. Grunau, C., Clark, S. J., and Rosenthal, A. Bisulfite genomic sequencing: systematic investigation of critical experimental parameters. Nucleic Acids Res, 29: E65-65., 2001.
9. Wang, R. Y., Kuo, K. C., Gehrke, C. W., Huang, L. H., and Ehrlich, M. Heat- and alkali-induced deamination of 5-methylcytosine and cytosine residues in DNA. Biochim Biophys Acta, 697: 371-377., 1982.
10. Herman, J. G., Graff, J. R., Myohanen, S., Nelkin, B. D., and Baylin, S. B. Methylation-specific PCR: a novel PCR assay for methylation status of CpG islands. Proc Natl Acad Sci USA, 93:9821-9826,1996.
11. Eads, C. A., Danenberg, K. D., Kawakami, K., Saltz, L. B., Blake, C., Shibata, D., Danenberg, P. V., and Laird, P. W. MethyLight: a high-throughput assay to measure DNA methylation. Nucleic Acids Res, 28: E32, 2000.
12. Wang, R. Y., Gehrke, C. W., and Ehrlich, M. Comparison of bisulfite modification of 5-methyldeoxycytidine and deoxycytidine residues. Nucleic Acids Res, 8:4777-4790.,1980.
13. Wong, I. H., Lo, Y. M., Zhang, J., Liew, C. T., Ng, M. H., Wong, N., Lai, P. B., Lau, W. Y., Hjelm, N. M., and Johnson, P. J. Detection of aberrant p16 methylation in the plasma and serum of liver cancer patients. Cancer Res, 59: 71-73, 1999.
14. Esteller, M., Sanchez-Cespedes, M., Rosell, R., Sidransky, D., Baylin, S. B., and Herman, J. G. Detection of aberrant promoter hypermethylation of tumor suppressor genes in serum DNA from non-small cell lung cancer patients [published erratum appears in Cancer Res 1999 Aug 1;59(15):3853]. Cancer Res, 59: 67-70, 1999.
15. Carlow, D. C., Carter, C. W., Jr., Mejlhede, N., Neuhard, J., and Wolfenden, R. Cytidine deaminases from B. subtilis and E. coli: compensating effects of changing zinc coordination and quaternary structure. Biochemistry, 38:12258-12265., 1999.
16. Vincenzetti, S., Cambi, A., Neuhard, J., Garattini, E., and Vita, A. Recombinant human cytidine deaminase: expression, purification, and characterization. Protein Expr Purif, 8: 247-253., 1996.
17. Sakai, T., Yu, T. S., Taniguchi, K., and Omata, S. Purification of cytosine deaminase from Pseudomonas aureofaciens. Agriculture and Biological Chemistry, 39: 2015-2020,1975.
18. Ipata, P. L., Marmocchi, F., Magni, G., Felicioli, R., and Polidoro, G. Baker's yeast cytosine deaminase. Some enzymic properties and allosteric inhibition by nucleosides and nucleotides. Biochemistry, 10: 4270-4276., 1971.
19. Yu, T. S., Kim, J. K., Katsuragi, T., Sakai, T., and Tonomura, K. Purification and properties of cytosine deaminase from Aspergillus-fumigatus. Journal of Fermentation and Bioengineering, 72: 266-269, 1991.
20. Teng, B., Burant, C. F., and Davidson, N. O. Molecular cloning of an apolipoprotein B messenger RNA editing protein. Science, 260: 1816-1819.,1993.
21. Powell, L. M., Wallis, S. C., Pease, R. J., Edwards, Y. H., Knott, T. J., and Scott, J. A novel form of tissue-specific RNA processing produces apolipoprotein-B48 in intestine. Cell, 50: 831-840., 1987.
22. Yamanaka, S., Balestra, M. E., Ferrell, L. D., Fan, J., Arnold, K. S., Taylor, S., Taylor, J. M., and Innerarity, T. L. Apolipoprotein B mRNA-editing protein induces hepatocellular carcinoma and dysplasia in transgenic animals. Proc Natl Acad Sci USA, 92: 8483-8487., 1995.
23. Harris, R. S., Petersen-Mahrt, S. K., and Neuberger, M. S. RNA editing enzyme APOBEC1 and some of its homologs can act as DNA mutators. Mol Cell, 10: 1247-1253., 2002.
24. Petersen-Mahrt, S. K. and Neuberger, M. S. In Vitro Deamination of Cytosine to Uracil in Single-stranded DNA by Apolipoprotein B Editing Complex Catalytic Subunit 1 (APOBEC1). J Biol Chem, 278: 19583-19586., 2003.
25. Diaz, M. and Storb, U. A novel cytidine deaminase AIDs in the delivery of error-prone polymerases to immunoglobulin genes. DNA Repair (Amst), 2: 623-627., 2003.
26. Dickerson, S. K., Market, E., Besmer, E., and Papavasiliou, F. N. AID Mediates Hypermutation by Deaminating Single Stranded DNA. J Exp Med, 197: 1291-1296., 2003.
27. Bransteitter, R., Pham, P., Scharff, M. D., and Goodman, M. F. Activation-induced cytidine deaminase deaminates deoxycytidine on single-stranded DNA but requires the action of RNase. Proc Natl Acad Sci U S A, 100: 4102-4107., 2003.
28. Chaudhuri, J., Tian, M., Khuong, C., Chua, K., Pinaud, E., and Alt, F. W. Transcription-targeted DNA deamination by the AID antibody diversification enzyme. Nature, 422: 726-730., 2003.
29. Sohail, A., Klapacz, J., Samaranayake, M., Ullah, A., and Bhagwat, A. S. Human activation-induced cytidine deaminase causes transcription-dependent, strand-biased C to U deaminations. Nucleic Acids Res, 31: 2990-2994., 2003.
30. Petersen-Mahrt, S. K., Harris, R. S., and Neuberger, M. S. AID mutates E. coli suggesting a DNA deamination mechanism for antibody diversification. Nature, 418:99-103., 2002.
31. Pham, P., Bransteitter, R., Petruska, J., and Goodman, M. F. Processive AID-catalysed cytosine deamination on single-stranded DNA simulates somatic hypermutation. Nature advance online publication, 18 June 2003 *(doi:10.1038*/*nature01760),* 2003. (Nature 424(no 6944):103-107, 2003)
32. Rehbein, H., Mackie, I. M., Pryde, S., Gonzales-Sotelo, C., Perez-Martin, R., Quinteiro, J., and Rey-Mendez, M. Comparison of different methods to produce single-strand DNA for identification of canned tuna by single-strand conformation polymorphism analysis. Electrophoresis, 19: 1381-1384., 1998.
33. Pant, K., Karpel, R. L., and Williams, M. C. Kinetic regulation of single DNA molecule denaturation by T4 gene 32 protein structural domains. J Mol Biol, 327: 571-578., 2003.
34. Villalva, C., Touriol, C., Seurat, P., Trempat, P., Delsol, G., and Brousset, P. Increased yield of PCR products by addition of T4 gene 32 protein to the SMART PCR cDNA synthesis system. Biotechniques, 31: 81-83, 86., 2001.
35. Guo, L. H. and Wu, R. New rapid methods for DNA sequencing based in exonuclease III digestion followed by repair synthesis. Nucleic Acids Res, 10: 2065-2084., 1982.
36. Puapaiboon, U., Jai-nhuknan, J., and Cowan, J. A. Rapid and direct sequencing of double-stranded DNA using exonuclease III and MALDI-TOF MS. Anal Chem, 72: 3338-3341., 2000.
37. Ward, R., Hawkins, N., O'Grady, R., Sheehan, C., O'Connor, T., Impey, H., Roberts, N., Fuery, C., and Todd, A. Restriction endonuclease-mediated selective polymerase chain reaction: a novel assay for the detection of K-ras mutations in clinical samples. Am J Pathol, 153: 373-379, 1998.
38. Sadri, R. and Hornsby, P. J. Rapid analysis of DNA methylation using new restriction enzyme sites created by bisulfite modification. Nucleic Acids Res, 24: 5058-5059, 1996.
39. Twyman R.M. Recombinant DNA and molecular cloning. Chapter 24. In: Advanced Molecular Biology: A Concise Reference. BIOS Scientific Publishers Limited.
40. Komiyama M et al JACS. 125, 3758 - 3762, 2003.
41. Izvolsky K et al Biochemistry 39, 10908-10913, 2000.
42. Neilsen P et al. Curr Opin Biotechnology 10, 71-75,1999.
43. Nielson P et al. Science 254,1497-1500,1991.
44. Kuhn H et al. JACS 124, 1097-1103, 2002.
45. Kuhn H et al. Nucleic Acid Research, 26, 582-287,1998.
46. Dennis Lo, Y.M., Wong, I.H.N., Zhang, Z., Tein, M.S.C., Ng, M.H.L. and Magnus Hjelm, N. Quantitative Analysis of aberrant p16 Methylation using real -time quantitive methylation-specific polymerase chain reaction" Cancer Research 59, 3899-3903,1999.

## Claims

1. A method for detecting the presence or level of alkylated cytosine in a sample of genomic or mitochondrial double stranded DNA from an individual, the method comprising:
(a) converting at least one region of the double stranded DNA to single stranded DNA;
(b) reacting a target region of the single stranded DNA from step (a) with at least one enzyme having cytidine deaminase activity, the enzyme differentially modifying alkylated cytosine and cytosine; and
(c) determining the level of enzymatic modification of the target region by the enzyme.

2. A method according to claim 1 wherein the single stranded DNA is reacted with the enzyme under conditions such that the enzyme reacts substantially only with either alkylated cytosine or cytosine in the single stranded DNA but not both.

3. A method according to claim 1 wherein the enzyme is capable of reacting substantially with only one of alkylated cytosine or cytosine in the single stranded DNA.

4. A method according to any one of claims 1 to 3 wherein the conversion of the region of the double stranded DNA to single stranded DNA comprises at least partially separating the two strands of the double stranded DNA.

5. A method according to claim 4 wherein one or more of strand displacing probes are utilised to at least partially separate the two strands of the double stranded DNA.

6. A method according to claim 5 wherein the or each strand displacing probe is independently selected from the group consisting of nucleic acid analogue probes, PNA containing probes, LNA containing probes, PNA probes and LNA probes.

7. A method according to claim 4 further comprising inhibiting annealing of the two strands of the double stranded DNA together once they have been separated to facilitate access to the target region by the enzyme.

8. A method according to claim 7 further comprising hybridising at least one probe with a strand of the double stranded DNA following separation of the two strands to thereby inhibit the annealing of the two strands together.

9. A method according to claim 8 wherein the at least one probe is independently selected from the group consisting of sense probes, looping probes, antisense probes and mixtures thereof.

10. A method according to claim 8 wherein at least two said probes are hybridised with the strand of the double stranded DNA, one of the probes hybridising with a region of the strand downstream of the target region and a further of the probes hybridising with a region of the strand upstream of the target region.

11. A method according to claim 8 wherein the probe hybridises with upstream and downstream regions of the strand which flank the target region such that a loop or bubble which incorporates the target region is formed in the strand.

12. A method according to claim 8 wherein the probe hybridises with the strand of the double stranded DNA either side of the target region and the probe has a middle region of non-complementary sequence that does not hybridise with the target region such that a loop or bubble incorporating the target region is formed in the strand.

13. A method according to claim 12 wherein the middle region of the probe incorporates inverted repeats that hybridise together following hybridisation of the probe with the strand of the double stranded DNA.

14. A method according to any one of claims 1 to 13 wherein the determination of the level of enzymatic modification of the single stranded DNA comprises analysing for sequence variations arising from the enzymatic modification of the target region of the single stranded DNA by the enzyme.

15. A method according to claim 14 wherein the determination of the level of enzymatic modification comprises subjecting the target region of the single stranded DNA to an amplification process involving thermocycling and primers to obtain an amplified product, and analysing the amplified product for sequence variations.

16. A method according to claim 15 wherein the analysis of the amplified product comprises subjecting the amplified product to a technique selected from the group consisting of nucleic acid sequencing, polymerase chain reaction techniques, restriction enzyme digests and techniques involving the use of probes that bind to specific nucleic acid sequences.

17. A method according to claim 16 wherein the analysis of the amplified product comprises subjecting the amplified product to a polymerase chain reaction technique.

18. A method according to any one of claims 1 to 17 wherein the at least one enzyme deaminates alkylated cytosine or cytosine in the target region of the single stranded DNA.

19. A method according to any one of claims 1 to 18 wherein a combination of different said enzymes are employed to differentially modify alkylated cytosine and cytosine in the target region.

20. A method according to any one of claims 1 to 19 wherein the or each enzyme is independently a deaminase enzyme or a catalytic fragment, variant, homologue, or a modified form or mutant form thereof, having deaminase activity of the enzyme.

21. A method according to claim 20, wherein the enzyme is selected from the group consisting of ApoBRe, AID, and AID mutant R35E/R36D.

22. A method according to any one of claims 1 to 21 comprising detecting the presence or level of alkylated cytosine in a gene or a non-coding region of a gene, or a fragment thereof.

23. A method according to claim 22 comprising detecting the presence or level of alkylated cytosine in a 5' untranslated region of a gene.

24. A method according to claim 23 wherein the level of alkylated cytosine comprises hypermethylation.

25. A method according to claim 23 wherein the level of alkylated cytosine comprises hypomethylation.

26. A method according to claim 23 wherein the gene is selected from the group consisting of *p16, E-cadherin,* the VHL gene, *BRCA1, p15,* hMLH1, ER, HIC1, MDG1, GST-π, O⁶-MGMT, calcitonin, *myo-D,* urokinase and S100A4.

27. A method according to any one of claims 1 to 26 wherein the detection of an altered level of alkylated cytosine in the target region of the single stranded DNA is a marker for a disease or condition.

28. A method according to claim 27 wherein the disease or condition is cancer.

29. A method according to claim 28 wherein the cancer is selected from the group consisting of lung cancer, breast cancer, colon cancer, bladder cancer, liver cancer, head and neck tumours, prostate cancer, renal cell tumours, leukemias, Burkitt lymphomas, brain tumours and carcinoma.

30. A method according to any one of the claims 1 to 23 further comprising diagnosing a disease or condition in the individual on the basis of the presence or the level of alkylated cytosine in the target region of the single stranded DNA.

31. A method according to claim 30 wherein the disease or condition comprises a cancer selected from the group consisting of lung cancer, breast cancer, colon cancer, bladder cancer, liver cancer, head and neck tumours, prostate cancer, renal cell tumours, leukemias, Burkitt lymphomas, brain tumours and carcinoma.

32. A method according to any one of claims 1 to 23 wherein the presence or level of the alkylated cytosine is detected to indicate the presence or absence of foetal DNA.

33. A method according to any one of claims 1 to 23 wherein the presence or level of the alkylated cytosine is detected for indicating the presence or absence of an altered gene imprinting state.

34. A method according to any one of claims 1 to 33 wherein the presence or level of the alkylated cytosine is detected to indicate the presence or absence of a pathogen or microorganism.

35. A method according to any one of claims 1 to 34 wherein the alkylated cytosine is methylated cytosine.

36. A method according to claim 35 wherein the methylated cytosine is 5-methylcytosine.

37. A method according to any one of claims 1 to 36 wherein the double stranded DNA is genomic DNA.

## Patentansprüche

1. Verfahren zur Detektion des Vorhandenseins oder der Menge von alkyliertem Cytosin in einer Probe aus genomischer oder mitochondrialer doppelsträngiger DNA von einem Individuum, wobei das Verfahren die folgenden Stufen umfasst:
(a) Umwandeln von mindestens einer Region der doppelsträngigen DNA in einzelsträngige DNA;
(b) Umsetzen einer Zielregion der einzelsträngigen DNA von Stufe (a) mit mindestens einem Enzym mit Cytidindeaminaseaktivität, wobei das Enzym alkyliertes Cytosin und Cytosin unterschiedlich modifiziert; und
(c) Bestimmen des Grades der enzymatischen Modifikation der Zielregion durch das Enzym.

2. Verfahren nach Anspruch 1, wobei die einzelsträngige DNA mit dem Enzym unter derartigen Bedingungen umgesetzt wird, dass das Enzym im wesentlichen nur mit entweder alkyliertem Cytosin oder Cytosin in der einzelsträngigen DNA, jedoch nicht mit beiden reagiert.

3. Verfahren nach Anspruch 1, wobei das Enzym fähig ist, im wesentlichen mit nur einer Spezies von alkyliertem Cytosin oder Cytosin in der einzelsträngigen DNA zu reagieren.

4. Verfahren nach einem der Ansprüche 1 bis 3, wobei die Umwandlung der Region der doppelsträngigen DNA in einzelsträngige DNA eine mindestens partielle Trennung der zwei Stränge der doppelsträngigen DNA umfasst.

5. Verfahren nach Anspruch 4, wobei eine oder mehrere Strangverdrängungssonden zur mindestens partiellen Trennung der zwei Stränge der doppelsträngigen DNA verwendet werden.

6. Verfahren nach Anspruch 5, wobei die Strangverdrängungssonde oder jede Strangverdrängungssonde unabhängig voneinander aus der Gruppe von Nucleinsäureanalogonsonden, PNA enthaltenden Sonden, LNA enthaltenden Sonden, PNA-Sonden und LNA-Sonden ausgewählt ist.

7. Verfahren nach Anspruch 4, das ferner das Hemmen des Zusammenlagerns der zwei Stränge der doppelsträngigen DNA, nachdem sie getrennt wurden, zum Erleichtern eines Zugangs zur Zielregion durch das Enzym umfasst.

8. Verfahren nach Anspruch 7, das ferner das Hybridisieren von mindestens einer Sonde mit einem Strang der doppelsträngigen DNA nach der Trennung der zwei Stränge, um dadurch das Zusammenlagern der zwei Stränge zu hemmen, umfasst.

9. Verfahren nach Anspruch 8, wobei die mindestens eine Sonde unabhängig voneinander aus der Gruppe von Sense-Sonden, Schleifenbildungssonden, Antisense-Sonden und Gemischen derselben ausgewählt ist.

10. Verfahren nach Anspruch 8, wobei eine Hybridisierung von mindestens zwei Sonden mit dem Strang der doppelsträngigen DNA erfolgt, wobei eine Sonde mit einer zur Zielregion strangabwärtigen Strangregion hybridisiert und eine weitere der Sonden mit einer zur Zielregion strangaufwärtigen Strangregion hybridisiert.

11. Verfahren nach Anspruch 8, wobei die Sonde mit strangaufwärtigen und strangabwärtigen Strangregionen, die die Zielregion flankieren, derart hybridisiert, dass in dem Strang eine die Zielregion enthaltende Schleife oder Blase gebildet wird.

12. Verfahren nach Anspruch 8, wobei die Sonde mit dem Strang der doppelsträngigen DNA von beiden Seiten der Zielregion hybridisiert und die Sonde einen Mittelbereich einer nichtkomplementären Sequenz, die mit der Zielregion nicht hybridisiert, derart aufweist, dass in dem Strang eine die Zielregion enthaltende Schleife oder Blase gebildet wird.

13. Verfahren nach Anspruch 12, wobei der Mittelbereich der Sonde invertierte Repeats enthält, die nach der Hybridisierung der Sonde mit dem Strang der doppelsträngigen DNA zusammenhybridisieren.

14. Verfahren nach einem der Ansprüche 1 bis 13, wobei die Bestimmung des Grades der enzymatischen Modifikation der einzelsträngigen DNA eine Analyse auf Sequenzvariationen, die aufgrund der enzymatischen Modifikation der Zielregion der einzelsträngigen DNA durch das Enzym auftreten, umfasst.

15. Verfahren nach Anspruch 14, wobei die Bestimmung des Grades der enzymatischen Modifikation das Durchführen eines Amplifikationsverfahrens an der Zielregion der einzelsträngigen DNA, das Thermocycling und Primer zur Gewinnung eines Amplifikationsprodukts umfasst, und das Analysieren des Amplifikationsprodukts auf Sequenzvariationen umfasst.

16. Verfahren nach Anspruch 15, wobei die Analyse des Amplifikationsprodukts das Durchführen einer Technik an dem Amplifikationsprodukt umfasst, die aus der Gruppe von Nucleinsäuresequenzierung, Polymerasekettenreaktionstechniken, Restriktionsenzymverdaus und Techniken, die die Verwendung von Sonden, die an spezifische Nucleinsäuersequenzen binden, umfassen, ausgewählt ist.

17. Verfahren nach Anspruch 16, wobei die Analyse des Amplifikationsprodukts das Durchführen einer Polymerasekettenreaktionstechnik an dem Amplifikationsprodukt umfasst.

18. Verfahren nach einem der Ansprüche 1 bis 17, wobei das mindestens eine Enzym alkyliertes Cytosin oder Cytosin in der Zielregion der einzelsträngigen DNA deaminiert.

19. Verfahren nach einem der Ansprüche 1 bis 18, wobei eine Kombination unterschiedlicher Enzyme zur selektiven Modifizierung von alkyliertem Cytosin und Cytosin in der Zielregion verwendet wird.

20. Verfahren nach einem der Ansprüche 1 bis 19, wobei das Enzym oder jedes der Enzyme unabhängig voneinander ein Deaminaseenzym oder ein katalytisches Fragment, eine Variante, ein Homologon oder eine modifizierte Form oder Mutantenform hiervon ist, die die Deaminaseaktivität des Enzyms aufweisen.

21. Verfahren nach Anspruch 20, wobei das Enzym aus der Gruppe von ApoBRe, AID und der AID-Mutante R35E/R36D ausgewählt ist.

22. Verfahren nach einem der Ansprüche 1 bis 21, das das Detektieren des Vorhandenseins oder der Menge von alkyliertem Cytosin in einem Gen oder einer nichtcodierenden Region eines Gens oder eines Fragments hiervon umfasst.

23. Verfahren nach Anspruch 22, das die Detektion des Vorhandenseins oder der Menge von alkyliertem Cytosin in einer 5'-nichttranslatierten Region eines Gens umfasst.

24. Verfahren nach Anspruch 23, wobei die Menge von alkyliertem Cytosin eine Hypermethylierung umfasst.

25. Verfahren nach Anspruch 23, wobei die Menge von alkyliertem Cytosin eine Hypomethylierung umfasst.

26. Verfahren nach Anspruch 23, wobei das Gen aus der Gruppe von p16, E-Cadherin, dem VHL-Gen, BRCA1, p15, hMLH1, ER, HICl, MDG1, GST-π, O⁶-MGMT, Calcitonin, myo-D, Urokinase und S100A4 ausgewählt ist.

27. Verfahren nach einem der Ansprüche 1 bis 26, wobei die Detektion einer veränderten Menge von alkyliertem Cytosin in der Zielregion der einzelsträngigen DNA ein Marker für eine Erkrankung oder einen Zustand ist.

28. Verfahren nach Anspruch 27, wobei die Erkrankung oder der Zustand eine Krebserkrankung ist.

29. Verfahren nach Anspruch 28, wobei die Krebserkrankung aus der Gruppe von Lungenkrebs, Brustkrebs, Kolonkrebs, Blasenkrebs, Leberkrebs, Kopf- und Nackentumoren, Prostatakrebs, Nierenzelltumoren, Leukämien, Burkitt-Lymphomen, Hirntumoren und Karzinom ausgewählt ist.

30. Verfahren nach einem der Ansprüche 1 bis 23, das ferner die Diagnose einer Erkrankung oder eines Zustands bei einem Individuum auf der Basis des Vorhandenseins oder der Menge von alkyliertem Cytosin in der Zielregion der einzelsträngigen DNA umfasst.

31. Verfahren nach Anspruch 30, wobei die Erkrankung oder der Zustand eine Krebserkrankung umfasst, die aus der Gruppe von Lungenkrebs, Brustkrebs, Kolonkrebs, Blasenkrebs, Leberkrebs, Kopf- und Nackentumoren, Prostatakrebs, Nierenzelltumoren, Leukämien, Burkitt-Lymphomen, Hirntumoren und Karzinom ausgewählt ist.

32. Verfahren nach einem der Ansprüche 1 bis 23, wobei das Vorhandensein oder die Menge von alkyliertem Cytosin detektiert wird, um das Vorhandensein oder Nichtvorhandensein von fetaler DNA anzuzeigen.

33. Verfahren nach einem der Ansprüche 1 bis 23, wobei das Vorhandensein oder die Menge von alkyliertem Cytosin detektiert wird, um das Vorhandensein oder Nichtvorhandensein eines Prägungszustands eines veränderten Gens anzuzeigen.

34. Verfahren nach einem der Ansprüche 1 bis 33, wobei das Vorhandensein oder die Menge von alkyliertem Cytosin detektiert wird, um das Vorhandensein oder Nichtvorhandensein von einem Pathogen oder Mikroorganismus anzuzeigen.

35. Verfahren nach einem der Ansprüche 1 bis 34, wobei das alkylierte Cytosin methyliertes Cytosin ist.

36. Verfahren nach Anspruch 35, wobei das methylierte Cytosin 5-Methylcytosin ist.

37. Verfahren nach einem der Ansprüche 1 bis 36, wobei die doppelsträngige DNA genomische DNA ist.

## Revendications

1. Procédé pour détecter la présence ou le niveau de cytosine alkylée dans un échantillon d'ADN bicaténaire génomique ou mitochondrial d'un individu, le procédé comprenant les étapes consistant à :
(a) convertir au moins une région de l'ADN bicaténaire en ADN monocaténaire ;
(b) faire réagir une région cible de l'ADN monocaténaire issu de l'étape (a) avec au moins une enzyme ayant une activité cytidine désaminase, l'enzyme modifiant différemment la cytosine alkylée et la cytosine ; et
(c) déterminer le niveau de modification enzymatique par l'enzyme de la région cible.

2. Procédé selon la revendication 1, dans lequel on fait réagir l'ADN monocaténaire avec l'enzyme dans des conditions telles que l'enzyme réagit pratiquement seulement avec la cytosine alkylée ou la cytosine dans l'ADN monocaténaire, mais pas avec les deux.

3. Procédé selon la revendication 1, dans lequel l'enzyme est capable de réagir pratiquement avec une seule parmi la cytosine alkylée et la cytosine dans l'ADN monocaténaire.

4. Procédé selon l'une quelconque des revendications 1 à 3, dans lequel la conversion de la région de l'ADN bicaténaire en ADN monocaténaire comprend la séparation au moins partielle des deux brins de l'ADN bicaténaire.

5. Procédé selon la revendication 4, dans lequel une ou plusieurs sondes déplaçant les brins sont utilisées pour séparer au moins partiellement les deux brins de l'ADN bicaténaire.

6. Procédé selon la revendication 5, dans lequel la ou les sondes déplaçant les brins sont indépendamment choisies dans le groupe consistant en sondes d'analogues d'acide nucléique, sondes contenant des PNA, sondes contenant des LNA, sondes de PNA et sondes de LNA.

7. Procédé selon la revendication 4, comprenant en outre l'inhibition de l'hybridation des deux brins de l'ADN bicaténaire ensemble une fois qu'ils ont été séparés, afin de faciliter l'accès de l'enzyme à la région cible.

8. Procédé selon la revendication 7, comprenant en outre l'hybridation d'au moins une sonde avec un brin de l'ADN bicaténaire après séparation des deux brins pour inhiber ainsi l'hybridation des deux brins ensemble.

9. Procédé selon la revendication 8, dans lequel la ou les sondes sont indépendamment choisies dans le groupe consistant en sondes sens, sondes à boucle, sondes antisens et leurs mélanges.

10. Procédé selon la revendication 8, dans lequel au moins deux desdites sondes sont hybridées avec le brin de l'ADN bicaténaire, une des sondes s"hybridant avec une région du brin en aval de la région cible et une autre des sondes s'hybridant avec une région du brin en amont de la région cible.

11. Procédé selon la revendication 8, dans lequel la sonde s'hybride avec des régions amont et avale du brin qui flanquent la région cible, de telle sorte qu'une boucle ou une bulle qui incorpore la région cible se forme dans le brin.

12. Procédé selon la revendication 8, dans lequel la sonde s'hybride avec le brin de l'ADN bicaténaire de chaque côté de la région cible et la sonde comprend une région médiane de séquence non complémentaire qui ne s'hybride pas avec la région cible, de telle sorte qu'une boucle ou une bulle incorporant la région cible se forme dans le brin.

13. Procédé selon la revendication 12, dans lequel la région médiane de la sonde incorpore des répétitions inversées qui s'hybrident ensemble après hybridation de la sonde avec le brin de l'ADN bicaténaire.

14. Procédé selon l'une quelconque des revendications 1 à 13, dans lequel la détermination du niveau de modification enzymatique de l'ADN monocaténaire comprend l'analyse des variations de séquence engendrées par la modification enzymatique par l'enzyme de la région cible de l'ADN monocaténaire.

15. Procédé selon la revendication 14, dans lequel la détermination du niveau de modification enzymatique comprend la soumission de la région cible de l'ADN monocaténaire à un processus d'amplification impliquant l'application d'un cycle thermique et des amorces, pour obtenir un produit amplifié, et l'analyse du produit amplifié pour détecter des variations de séquences.

16. Procédé selon la revendication 15, dans lequel l'analyse du produit amplifié comprend la soumission du produit amplifié à une technique choisie dans le groupe consistant en séquençage d'acide nucléique, techniques de réaction en chaîne de polymérase, digestions par enzymes de restriction et techniques impliquant l'utilisation de sondes qui se lient à des séquences d'acide nucléique spécifiques.

17. Procédé selon la revendication 16, dans lequel l'analyse du produit amplifié comprend la soumission du produit amplifié à une technique de réaction en chaîne de polymérase.

18. Procédé selon l'une quelconque des revendications 1 à 17, dans lequel la ou les enzymes désaminent la cytosine alkylée ou la cytosine dans la région cible de l'ADN monocaténaire.

19. Procédé selon l'une quelconque des revendications 1 à 18, dans lequel une combinaison de dites enzymes différentes est employée pour modifier différemment la cytosine alkylée et la cytosine dans la région cible.

20. Procédé selon l'une quelconque des revendications 1 à 19, dans lequel l'enzyme ou chaque enzyme est indépendamment une enzyme désaminase ou un fragment catalytique, variant, homologue ou une forme modifiée ou une forme mutante de celle-ci, ayant l'activité désaminase de l'enzyme.

21. Procédé selon la revendication 20, dans lequel l'enzyme est choisie dans le groupe consistant en ApoBRe, AID, et le mutant d'AID R35E/R36D.

22. Procédé selon l'une quelconque des revendications 1 à 21, comprenant la détection de la présence ou du niveau de cytosine alkylée dans un gène ou une région non codante d'un gène ou un fragment de ceux-ci.

23. Procédé selon la revendication 22, comprenant la détection de la présence ou du niveau de cytosine alkylée dans une région 5' non traduite du gène.

24. Procédé selon la revendication 23, dans lequel le niveau de cytosine alkylée comprend l'hyperméthylation.

25. Procédé selon la revendication 23, dans lequel le niveau de cytosine alkylée comprend l'hypométhylation.

26. Procédé selon la revendication 23, dans lequel le gène est choisi dans le groupe consistant en *p16, E-cadhérine,* le gène de VHL, *BRCA1, p15,* hMLH1, ER, H1C1, MDG1, GST-π, O⁶-MGMT, calcitonine, *myo-D,* urokinase et S100A4.

27. Procédé selon l'une quelconque des revendications 1 à 26, dans lequel la détection d'un niveau altéré de cytosine alkylée dans la région cible de l'ADN monocaténaire est un marqueur pour une maladie ou une condition.

28. Procédé selon la revendication 27, dans lequel la maladie ou la condition est un cancer.

29. Procédé selon la revendication 28, dans lequel le cancer est choisi dans le groupe consistant en cancer du poumon, cancer du sein, cancer du côlon, cancer de la vessie, cancer du foie, tumeurs de la tête et du cou, cancer de la prostate, cancer à cellules rénales, leucémies, lymphomes de Burkitt, tumeurs du cerveau et carcinome.

30. Procédé selon l'une quelconque des revendications 1 à 23, comprenant en outre le diagnostic d'une maladie ou d'une condition chez l'individu sur la base de la présence ou du niveau de cytosine alkylée dans la région cible de l'ADN monocaténaire.

31. Procédé selon la revendication 30, dans lequel la maladie ou la condition comprend un cancer choisi dans le groupe consistant en cancer du poumon, cancer du sein, cancer du côlon, cancer de la vessie, cancer du foie, tumeurs de la tête et du cou, cancer de la prostate, cancer à cellules rénales, leucémies, lymphomes de Burkitt, tumeurs du cerveau et carcinome.

32. Procédé selon l'une quelconque des revendications 1 à 23, dans lequel la présence ou le niveau de cytosine alkylée est détecté pour indiquer la présence ou l'absence d'ADN foetal.

33. Procédé selon l'une quelconque des revendications 1 à 23, dans lequel la présence ou le niveau de cytosine alkylée est détecté pour indiquer la présence ou l'absence d'un état d'empreinte d'un gène altéré.

34. Procédé selon l'une quelconque des revendications 1 à 33, dans lequel la présence ou le niveau de cytosine alkylée est détecté pour indiquer la présence ou l'absence d'un pathogène ou d'un microorganisme.

35. Procédé selon l'une quelconque des revendications 1 à 34, dans lequel la cytosine alkylée est la cytosine méthylée.

36. Procédé selon la revendication 35, dans lequel la cytosine méthylée est la 5-méthylcytosine.

37. Procédé selon l'une quelconque des revendications 1 à 36, dans lequel l'ADN bicaténaire est de l'ADN génomique.
